# EUROPÄISCHE PATENTSCHRIFT

(11) **EP 1 934 159 B1**
(45) Veröffentlichungstag und Bekanntmachung des Hinweises auf die Patenterteilung: **25.03.2009**
(21) Anmeldenummer: 06793925.6
(22) Anmeldetag: 29.09.2006
(51) Int. Cl.: C07B 57/00, C07C 29/80

(54) **VERFAHREN ZUR AUFTRENNUNG STEREOISOMERER VERBINDUNGEN**
METHOD FOR FRACTIONATING STEREOISOMERIC COMPOUNDS
PROCEDE DE SEPARATION DE COMPOSES STEREOISOMERES

(30) Priorität: 30.09.2005 DE 102005046917; 11.05.2006 EP 06113819
(43) Veröffentlichungstag der Anmeldung: 25.06.2008
(73) Patentinhaber: BASF SE, 67056 Ludwigshafen (DE)
(72) Erfinder: JÄKEL, Christoph, 67117 Limburgerhof (DE); PACIELLO, Rocco, 67098 Bad Dürkheim (DE); KASHANI-SHIRAZI, Nawid, 68549 Ilvesheim (DE); KAIBEL, Gerd, 68623 Lampertheim (DE)
(74) Vertreter: Reitstötter - Kinzebach
(86) Internationale Anmeldenummer: PCT/EP2006/066910
(87) Internationale Veröffentlichungsnummer: WO 2007/036570

(56) Entgegenhaltungen:
- EP-A- 0 970 936
- FR-A1- 2 176 621

## Beschreibung

Die vorliegende Erfindung betrifft ein Verfahren zur Auftrennung Verbindungen, die wenigstens eine Alkohol- und/oder Aminogruppe aufweisen.

Chirale Alkohole und Amine sind begehrte Einsatzstoffe, z. B. in der Feinchemie und der pharmazeutischen Chemie. Sie werden unter anderem in maßgeschneiderten Materialien mit nichtlinearen optischen Eigenschaften, als Geruchs- und Geschmacksstoffe oder als Vorstufen für Medikamente eingesetzt. Es besteht ein hohes Interesse an der möglichst kostengünstigen Bereitstellung chiraler Alkohole. Chirale Alkohole können auf verschiedene Arten hergestellt bzw. isoliert werden. Zum einen können sie direkt oder nach entsprechender chemischer Transformation aus natürlich zur Verfügung stehenden Verbindungen, dem sog. "Chiral Pool", erhalten werden. Des Weiteren besteht die Möglichkeit zur Herstellung chiraler Alkohole durch asymmetrische Synthese, d. h. eine Reaktion bei der die resultierenden stereoisomeren Produkte (Enantiomere oder Diastereomere) in ungleichen Mengen entstehen. Des Weiteren besteht die Möglichkeit chirale Alkohole, ausgehend von racemischen Alkoholen, durch eine chirale Diskriminierung im Sinne einer Racematspaltung zu erhalten. Eine solche chirale Diskriminierung ist dann besonders vorteilhaft, wenn der entsprechende racemische Alkohol einfach zugängig ist. Verschiedene Verfahren zur chiralen Diskriminierung von stereoisomeren Alkoholen sind bekannt. So können diese mittels Enzymen, Metallkomplexen oder kleinen organischen Molekülen als Katalysatoren einer kinetischen Racematspaltung im Sinne einer enantiodifferenzierenden Transformation unterzogen werden. Allerdings eignen sich die verwendeten Katalysatoren nur für eine stark eingeschränkte Anzahl von Alkoholen und/oder die hohen Gestehungskosten der Katalysatoren führen zu einer wirtschaftlichen Benachteiligung des Verfahrens. Des Weiteren ist bekannt, stereoisomere Alkohole durch adsorbtive Trennverfahren an optisch aktiven immobilisierten Phasen aufzutrennen. Zunehmende Bedeutung kommt dabei der SMB-Chromatographie (Simulated-Moving-Bed-Chromatographie) zu, die eine kontinuierliche Auftrennung von stereoisomeren Substanzgemischen in zwei verschiedene Fraktionen ermöglicht. Ein Nachteil dieser Technologie liegt in den relativ hohen Kosten der zu verwendenden chiralen stationären Phasen sowie in der Notwendigkeit der Verwendung von Lösungsmitteln, von denen die Stereoisomere in einem separaten Schritt abgetrennt werden müssen. Es ist weiterhin bekannt, racemische Verbindungen mit Hilfe chiraler Diskriminatoren (Selektoren) durch Destillation, speziell extraktive Rektifikation, aufzutrennen.

Die FR-A-2,176,621 beschreibt ein Verfahren zur Auftrennung optischer Isomeren durch Extraktivdestillation. Dazu gehört ganz allgemein der Einsatz von Diskriminatoren, die zur Bildung unterschiedlich stabiler Komplexe mit den optischen Antipoden befähigt sind, zu Erzielung eines Unterschiedes bezüglich einer physikalischen Eigenschaft, speziell des Siedepunkts.

Die EP-A-0970936 beschreibt ein Verfahren zur Auftrennung optischer Isomeren, bei dem man diese in einem Gegenstromverfahren mit einer Diskriminierungsflüssigkeit, die ein Diskriminierungsmittel enthält, und einem Verdünnungsmittel in Kontakt bringt und einer Auftrennung der optischen Isomere durch adsorbtive Trennung, destillative Trennung oder Membrantrennung unterzieht. Als chirale Diskriminatoren werden Saccharide und Saccharidderivate, insbesondere Cyclodextrine und Cyclodextrinderivate eingesetzt. Nachteilig an diesen Diskriminatoren ist, dass sie in aufwendiger Synthese aufgebaut werden müssen, was zu einer wirtschaftlichen Benachteiligung dieses Verfahrens führt.

Der vorliegenden Erfindung liegt die Aufgabe zugrunde, ein Verfahren zur Verfügung zu stellen, das eine möglichst wirtschaftliche Auftrennung stereoisomerer Verbindungen, die wenigstens eine Alkohol- und/oder Aminogruppe aufweisen, ermöglicht.

Demgemäß wurde ein Verfahren zur Auftrennung stereoisomerer Verbindungen, speziell Alkohole, durch destillative Trennung in Gegenwart eines chiralen Diskriminators gefunden, bei dem es sich um einen Metallkomplex handelt, der sich von einer Verbindung der allgemeinen Formel I 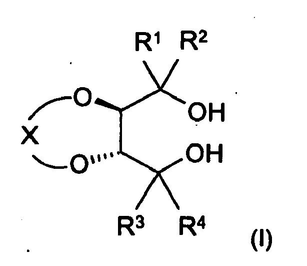 ableitet, worin
- R¹, R², R³ und R⁴: unabhängig voneinander für Alkyl, Cycloalkyl, Heterocycloalkyl, Aryl oder Hetaryl stehen, wobei die Alkylreste 1, 2, 3, 4 oder 5 Substituenten, ausgewählt unter Cycloalkyl, Heterocycloalkyl, Aryl, Hetaryl, Alkoxy, Cycloalkoxy, Heterocycloalkoxy, Aryloxy, Hetaryloxy, Hydroxy, Thiol, Polyalkylenoxid, Polyalkylenimin, COOH, Carboxylat, SO₃H, Sulfonat, NE¹E², NE¹E²E³A⁻, Halogen, Nitro, Acyl oder Cyano aufweisen können, worin E¹, E² und E³ jeweils gleiche oder verschiedene Reste, ausgewählt unter Wasserstoff, Alkyl, Cycloalkyl, oder Aryl bedeuten und A⁻ für ein Anionäquivalent steht,
und wobei die Cycloalkyl-, Heterocycloalkyl-, Aryl- und Hetarylreste R¹, R², R³ und R⁴ je 1, 2, 3, 4 oder 5 Substituenten aufweisen können, die ausgewählt sind unter Alkyl und den zuvor für die Alkylreste R¹ bis R⁴ genannten Substituenten, oder
- R¹ und R² und/oder R³ und R⁴: zusammen mit dem Kohlenstoffatom an das sie gebunden sind, für einen 5- bis 8-gliedrigen Cyclus stehen, der gegebenenfalls zusätzlich ein-, zwei- oder dreifach mit Cycloalkyl, Heterocycloalkyl, Aryl oder Hetaryl anelliert ist, wobei der Cyclus und, falls vorhanden, die anellierten Gruppen unabhängig voneinander je einen, zwei, drei oder vier Substituenten tragen können, die ausgewählt sind unter Alkyl, Cycloalkyl, Heterocycloalkyl, Aryl, Hetaryl, Hydroxy, Thiol, Polyalkylenoxid, Polyalkylenimin, Alkoxy, Halogen, COOH, Carboxylat, SO₃H, Sulfonat, NE⁴E⁵, NE⁴E⁵E⁶A⁻, Nitro, Alkoxycarbonyl, Acyl oder Cyano stehen, worin E⁴, E⁵ und E⁶ jeweils gleiche oder verschiedene Reste, ausgewählt unter Wasserstoff, Alkyl, Cycloalkyl und Aryl bedeuten und A⁻ für ein Anionäquivalent steht,
- X: zusammen mit den Sauerstoffatomen, an die es gebunden ist, für einen 5- bis 8-gliedrigen Heterocyclus steht, der gegebenenfalls ein-, oder zweifach mit Cycloalkyl, Heterocycloalkyl, Aryl und/oder Hetaryl anelliert ist, wobei die anellierten Gruppen unabhängig voneinander je einen, zwei, drei oder vier Substituenten, ausgewählt unter Alkyl, Cycloalkyl, Heterocycloalkyl, Aryl, Hetaryl, Hydroxy, Thiol, Polyalkylenoxid, Polyalkylenimin, Alkoxy, Halogen, COOH, Carboxylat, SO₃H, Sulfonat, NE⁷E⁸, NE⁷E⁸E⁹A⁻, Nitro, Alkoxycarbonyl, Acyl oder Cyano, worin E⁷, E⁸ und E⁹ jeweils gleiche oder verschiedene Reste, ausgewählt unter Wasserstoff, Alkyl, Cycloalkyl und Aryl bedeuten und A⁻ für ein Anionäquivalent steht, tragen können und/oder X einen, zwei, drei oder vier Substituenten aufweisen kann, die ausgewählt sind unter den zuvor für die anellierten Gruppen genannten Substituenten und/oder X durch 1 oder 2 gegebenenfalls substituierte Heteroatome unterbrochen sein kann.

"Chirale Verbindungen" sind im Rahmen der vorliegenden Erfindung Verbindungen mit wenigstens einem Chiralitätszentrum (z. B. wenigstens einem asymmetrischen Atom, insbesondere wenigstens einem asymmetrischen C-Atom oder P-Atom), mit Chiralitätsachse, Chiralitätsebene oder Schraubenwindung. Der Begriff "chiraler Diskriminator" umfasst Diskriminatoren, die wenigstens einen chiralen Liganden aufweisen. "Achirale Verbindungen" sind Verbindungen, die nicht chiral sind.

Eine "racemische Mischung" wird auch "Racemat" genannt und bezeichnet eine äquimolare Mischung zweier chemischer Verbindungen, die sich zu einander wie Bild und Spiegelbild verhalten.

Der Begriff "Racematspaltung" bezeichnet eine Trennung einer racemischen Mischung in Fraktionen, die die einzelnen Verbindungen, die sich zu einander wie Bild und Spiegelbild verhalten, in reiner oder zumindest angereicherter Form enthalten. Eine Racematspaltung im Sinne der Erfindung liegt daher auch dann vor, wenn Mischungen der Komponenten erhalten werden, die nicht äquimolar sind.

"Stereoisomere" sind Verbindungen gleicher Konstitution aber unterschiedlicher Atomanordnung im dreidimensionalen Raum.

"Enantiomere" sind Stereoisomere, die sich zueinander wie Bild zu Spiegelbild verhalten. Der bei einer asymmetrischen Synthese erzielte "Enantiomeren-Überschuss" (enantiomeric excess, ee) ergibt sich dabei nach folgender Formel: ee[%] = (R-S)/(R+S) x 100. R und S sind die Deskriptoren des CIP-Systems für die beiden Enantiomeren und geben die absolute Konfiguration am asymmetrischen Atom wieder. Die enantlomerenreine Verbindung (ee = 100 %) wird auch als "homochirale Verbindung" bezeichnet.

Das erfindungsgemäße Verfahren führt zu Produkten, die bezüglich eines bestimmten Stereoisomers angereichert sind. Der erzielte "Enantiomeren-Überschuss" (ee) beträgt in der Regel wenigstens 1 %.

"Diastereomere" sind Stereoisomere, die nicht enantiomer zueinander sind.

Im Folgenden umfasst der Ausdruck "Alkyl" geradkettige und verzweigte Alkylgruppen. Vorzugsweise handelt es sich dabei um geradkettige oder verzweigte C₁-C₂₀-Alkyl, bevorzugterweise C₁-C₁₂-Alkyl-, besonders bevorzugt C₁-C₈-Alkyl- und ganz besonders bevorzugt C₁-C₆-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl; 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

Der Ausdruck "Alkyl" umfasst auch substituierte Alkylgruppen, welche im Allgemeinen 1, 2, 3, 4 oder 5, bevorzugt 1, 2 oder 3 und besonders bevorzugt 1 Substituenten, ausgewählt aus den Gruppen Cycloalkyl, Aryl, Hetaryl, Halogen, NE¹E², NE¹E²E³⁺, COOH, Carboxylat, -SO₃H und Sulfonat, tragen können.

Der Ausdruck "Alkylen" im Sinne der vorliegenden Erfindung steht für geradkettige oder verzweigte Alkandiyl-Gruppen mit vorzugsweise 1 bis 6, insbesondere 1 bis 4 Kohlenstoffatomen. Dazu zählen Methylen (-CH₂-), Ethylen (-CH₂-CH₂-), n-Propylen (-CH₂-CH₂-CH₂-), Isopropylen (-CH₂-CH(CH₃)-) etc.

Der Ausdruck "Cycloalkyl" umfasst im Sinne der vorliegenden Erfindung unsubstituierte als auch substituierte Cycloalkylgruppen, vorzugsweise C₃-C₈-Cycloalkylgruppen, wie Cyclopentyl, Cyclohexyl oder Cycloheptyl, die im Falle einer Substitution, im Allgemeinen 1, 2, 3, 4 oder 5, bevorzugt 1, 2 oder 3 und besonders bevorzugt 1 Substituenten, vorzugsweise ausgewählt aus den für Alkyl genannten Substituenten, tragen können.

Der Ausdruck "Heterocycloalkyl" im Sinne der vorliegenden Erfindung umfasst gesättigte, cycloaliphatische Gruppen mit im Allgemeinen 4 bis 7, vorzugsweise 5 oder 6 Ringatomen, in denen 1 oder 2 der Ringkohlenstoffatome durch Heteroatome, vorzugsweise ausgewählt aus den Elementen Sauerstoff, Stickstoff und Schwefel, ersetzt sind und die gegebenenfalls substituiert sein können, wobei im Falle einer Substitution, diese heterocycloaliphatischen Gruppen 1, 2 oder 3, vorzugsweise 1 oder 2, besonders bevorzugt 1 Substituenten, ausgewählt aus Alkyl, Aryl, COOR^{f}, COO⁻M⁺ und NE¹E², bevorzugt Alkyl, tragen können. Beispielhaft für solche heterocycloaliphatischen Gruppen seien Pyrrolidinyl, Piperidinyl, 2,2,6,6-Tetramethylpiperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Tetrahydrofuranyl, Tetrahydropyranyl, Dioxanyl genannt.

Der Ausdruck "Aryl" umfasst im Sinne der vorliegenden Erfindung unsubstituierte als auch substituierte Arylgruppen, und steht vorzugsweise für Phenyl, Tolyl, Xylyl, Mesityl, Naphthyl, Fluorenyl, Anthracenyl, Phenanthrenyl oder Naphthacenyl, 4'-Biphenylyl, 3'-Biphenylyl, 2'-Biphenyl besonders bevorzugt für Phenyl, 4'-Biphenyl oder Naphthyl, wobei diese Arylgruppen im Falle einer Substitution im Allgemeinen 1, 2, 3, 4 oder 5, vorzugsweise 1, 2 oder 3 und besonders bevorzugt 1 Substituenten, ausgewählt aus den Gruppen Alkyl, Alkoxy, Carboxyl, Carboxylat, -SO₃H, Sulfonat, NE¹E², Alkylen-NE¹E², Nitro, Cyano oder Halogen, tragen können.

Der Ausdruck "Hetaryl" umfasst im Sinne der vorliegenden Erfindung unsubstituierte oder substituierte, heterocycloaromatische Gruppen, vorzugsweise die Gruppen Pyridyl, Chinolinyl, Acridinyl, Pyridazinyl, Pyrimidinyl, Pyrazinyl, Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl, wobei diese heterocycloaromatischen Gruppen im Falle einer Substitution im Allgemeinen 1, 2 oder 3 Substituenten, ausgewählt aus den Gruppen Alkyl, Alkoxy, Acyl, Carboxyl, Carboxylat, -SO₃H, Sulfonat, NE¹E², Alkylen-NE¹E² oder Halogen, tragen können.

Die obigen Erläuterungen zu den Ausdrücken "Alkyl", "Cycloalkyl", "Aryl", "Heterocycloalkyl" und "Hetaryl" gelten entsprechend für die Ausdrücke "Alkoxy", "Cycloalkoxy", "Aryloxy", "Heterocycloalkoxy" und "Hetaryloxy".

Der Ausdruck "Acyl" steht im Sinne der vorliegenden Erfindung für Alkanoyl- oder Aroylgruppen mit im Allgemeinen 2 bis 11, vorzugsweise 2 bis 8 Kohlenstoffatomen, beispielsweise für die Acetyl-, Propanoyl-, Butanoyl-, Pentanoyl-, Hexanoyl-, Heptanoyl-, 2-Ethylhexanoyl-, 2-Propylheptanoyl-, Benzoyl- oder Naphthoyl-Gruppe.

Die Reste E¹ bis E¹² sind unabhängig voneinander ausgewählt unter Wasserstoff, Alkyl, Cycloalkyl und Aryl. Die Gruppen NE¹E², NE⁴E⁵, NE⁷E⁸ und NE¹⁰E¹¹ stehen vorzugsweise für N,N-Dimethylamino, N-Ethyl-N-methylamino, N,N-Diethylamino, N,N-Dipropylamino, N,N-Diisopropylamino, N,N-Di-n-butylamino, N,N-Di-t.-butylamino, N,N-Dicyclohexylamino oder N,N-Diphenylamino.

Halogen steht für Fluor, Chlor, Brom und Iod, bevorzugt für Fluor, Chlor und Brom.

Unter einem Kationäquivalent wird ein einwertiges Kation oder der einer positiven Einfachladung entsprechende Anteil eines mehrwertigen Kations verstanden. Vorzugsweise werden Alkalimetall-, insbesondere Na⁺, K⁺-, Li⁺-lonen oder Onium-lonen, wie Ammonium-, Mono-, Di-, Tri-, Tetraalkylammonium-, Phosphonium-, Tetraalkylphosphonium- oder Tetraarylphosphonium-Ionen verwendet.

In einer bevorzugten Ausführungsform stehen in den Verbindungen der allgemeinen Formel I die Substituenten R¹ und R² und/oder R³ und R⁴ für Gruppen, die nicht miteinander verbunden sind. Bevorzugt R¹, R², R³ und R⁴ unabhängig voneinander ausgewählt sind unter Gruppen der Formeln II.a bis II.c 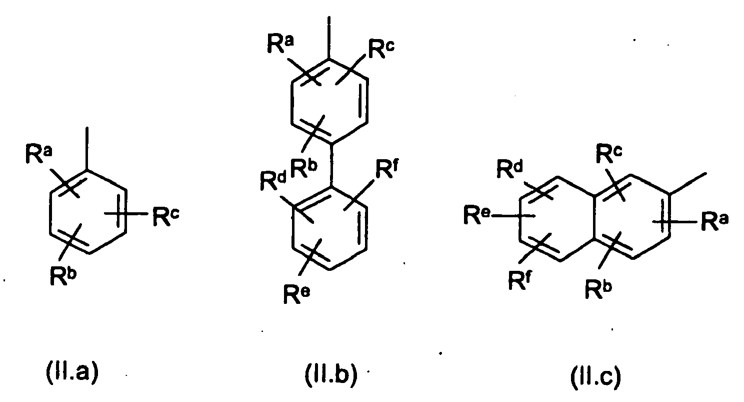
- R^{a}, R^{b}, R^{c}, R^{d}, R^{e} und R^{f}: unabhängig voneinander für Wasserstoff, C₁-C₄-Alkyl, C₁-C₄-Alkoxy, Acyl, Halogen, Trifluormethyl, C₁-C₄-Alkoxycarbonyl oder Carboxyl stehen.

In einer bevorzugten Ausführungsform sind die Reste R¹, R², R³ und R⁴ ausgewählt unter Resten der Formel II.a, worin R^{a}, R^{b} und R^{c} unabhängig voneinander für Wasserstoff, C₁-C₄-Alkyl, besonders bevorzugt Methyl, Ethyl, n-Propyl, Isopropyl, n-Butyl oder tert.-Butyl, C₁-C₄-Alkoxy, bevorzugt Methoxy, Ethoxy, n-Propoxy, Isopropoxy, n-Butoxy, tert.-Butoxy, Aryl, bevorzugt Phenyl, Halogen, bevorzugt Fluor oder Chlor, Trifluormethyl, Methoxycarbonyl oder Ethoxycarbonyl stehen. In einer bevorzugten Ausführung stehen R^{a}, R^{b} und R^{c} für Wasserstoff.

In einer weiteren bevorzugten Ausführungsform sind die Reste R¹, R², R³ und R⁴ ausgewählt unter Resten der Formel II.b, worin R^{a}, R^{b}, R^{c}, R^{d}, R^{e} und R^{f} unabhängig voneinander für Wasserstoff, C₁-C₄-Alkyl, besonders bevorzugt Methyl, Ethyl, n-Propyl, Isopropyl, n-Butyl oder tert.-Butyl, C₁-C₄-Alkoxy, bevorzugt Methoxy, Ethoxy, n-Propoxy, Isopropoxy, n-Butoxy, tert.-Butoxy, Halogen, bevorzugt Fluor oder Chlor, Trifluormethyl, Methoxycarbonyl oder Ethoxycarbonyl stehen. Bevorzugt sind einer, zwei oder drei der Reste R^{a} bis R^{f} unabhängig voneinander ausgewählt unter C₁-C₄-Alkyl, besonders bevorzugt Methyl, Ethyl, n-Propyl, Isopropyl, n-Butyl oder tert.-Butyl, C₁-C₄-Alkoxy, bevorzugt Methoxy, Ethoxy, n-Propoxy, Isopropoxy, n-Butoxy, tert.-Butoxy, Halogen, bevorzugt Fluor oder Chlor, Trifluormethyl, Methoxycarbonyl oder Ethoxycarbonyl, und die übrigen der Reste R^{a} bis R^{f} stehen für Wasserstoff. In einer bevorzugten Ausführung stehen R^{a}, R^{b}, R^{c}, R^{d}, R^{e} und R^{f} für Wasserstoff.

In einer weiteren bevorzugten Ausführungsform sind die Reste R¹, R², R³ und R⁴ ausgewählt unter Resten der Formel II.c, worin R^{a}, R^{b}, R^{c}, R^{d}, R^{e} und R^{f} unabhängig voneinander für Wasserstoff, C₁-C₄-Alkyl, besonders bevorzugt Methyl, Ethyl, n-Propyl, Isopropyl, n-Butyl oder tert.-Butyl, C₁-C₄-Alkoxy, bevorzugt Methoxy, Ethoxy, n-Propoxy, Isopropoxy, n-Butoxy, tert.-Butoxy, Halogen, bevorzugt Fluor oder Chlor, Trifluormethyl, Methoxycarbonyl oder Ethoxycarbonyl stehen. Bevorzugt sind einer, zwei oder drei der Reste R^{a} bis R^{f} unabhängig voneinander ausgewählt unter C₁-C₄-Alkyl, besonders bevorzugt Methyl, Ethyl, n-Propyl, Isopropyl, n-Butyl oder tert.-Butyl, C₁-C₄-Alkoxy, bevorzugt Methoxy, Ethoxy, n-Propoxy, Isopropoxy, n-Butoxy, tert.-Butoxy, Halogen, bevorzugt Fluor oder Chlor, Trifluormethyl, Methoxycarbonyl oder Ethoxycarbonyl, und die übrigen der Reste R^{a} bis R^{f} stehen für Wasserstoff. In einer bevorzugten Ausführung stehen R^{a}, R^{b}, R^{c}, R^{d}, R^{e} und R^{f} für Wasserstoff.

Besonders bevorzugt stehen R¹, R², R³ und R⁴ alle für Phenyl, alle für 4-Biphenylyl oder alle für 2-Naphthyl.

In einer weiteren bevorzugten Ausführungsform stehen R¹ und R² und/oder R³ und R⁴ zusammen mit dem Kohlenstoffatom an das sie gebunden sind, für einen 5- bis 8-gliedrigen Heterocyclus stehen, der gegebenenfalls zusätzlich ein-, zwei- oder dreifach mit Cycloalkyl, Heterocycloalkyl, Aryl oder Hetaryl anelliert ist, wobei der Heterocyclus und, falls vorhanden, die anellierten Gruppen unabhängig voneinander je einen, zwei, drei oder vier Substituenten tragen können, die ausgewählt sind unter Alkyl, Cycloalkyl, Heterocycloalkyl, Aryl, Hetaryl, Hydroxy, Thiol, Polyalkylenoxid, Polyalkylenimin, Alkoxy, Halogen, COOH, Carboxylat, SO₃H, Sulfonat, NE⁴E⁵, NE⁴E⁵E⁶A⁻, Nitro, Alkoxycarbonyl, Acyl oder Cyano stehen, worin E⁴, E⁵ und E⁶ jeweils gleiche oder verschiedene Reste, ausgewählt unter Wasserstoff, Alkyl, Cycloalkyl und Aryl bedeuten und A⁻ für ein Anionäquivalent steht.

Die verbrückende Gruppe X ist vorzugsweise ausgewählt unter Gruppen der Formeln C=O, C=S und CR⁵R⁶, worin
- R⁵ und R⁶: unabhängig voneinander für Alkyl, Cycloalkyl, Heterocycloalkyl, Aryl oder Hetaryl stehen, wobei die Alkylreste 1, 2, 3, 4 oder 5 Substituenten, ausgewählt unter Cycloalkyl, Heterocycloalkyl, Aryl, Hetaryl, Alkoxy, Cycloalkoxy, Heterocycloalkoxy, Aryloxy, Hetaryloxy, Hydroxy, Thiol, Polyalkylenoxid, Polyalkylenimin, COOH, Carboxylat, SO₃H, Sulfonat, NE¹⁰E¹¹, NE¹⁰E¹¹E¹²A⁻, Halogen, Nitro, Acyl oder Cyano aufweisen können, worin E¹⁰, E¹¹ und E¹² jeweils gleiche oder verschiedene Reste, ausgewählt unter Wasserstoff, Alkyl, Cycloalkyl, oder Aryl bedeuten und A⁻ für ein Anionäquivalent steht,
und wobei die Cycloalkyl-, Heterocycloalkyl-, Aryl- und Hetarylreste R⁵ und R⁶ je 1, 2, 3, 4 oder 5 Substituenten aufweisen können, die ausgewählt sind unter Alkyl und den zuvor für die Alkylreste R⁵ bis R⁶ genannten Substituenten, oder
- R⁵ und R⁶: zusammen mit dem Kohlenstoffatom an das sie gebunden sind, für einen 5- bis 8-gliedrigen Heterocyclus stehen, der gegebenenfalls zusätzlich ein-, zwei- oder dreifach mit Cycloalkyl, Heterocycloalkyl, Aryl oder Hetaryl anelliert ist, wobei der Heterocyclus und, falls vorhanden, die anellierten Gruppen unabhängig voneinander je einen, zwei, drei oder vier Substituenten tragen können, die ausgewählt sind unter Alkyl, Cycloalkyl, Heterocycloalkyl, Aryl, Hetaryl, Hydroxy, Thiol, Polyalkylenoxid, Polyalkylenimin, Alkoxy, Halogen, COOH, Carboxylat, SO₃H, Sulfonat NE¹³E¹⁴, NE¹³E¹⁴E¹⁵A⁻, Nitro, Alkoxycarbonyl, Acyl oder Cyano stehen, worin E¹³, E¹⁴ und E¹⁵ jeweils gleiche oder verschiedene Reste, ausgewählt unter Wasserstoff, Alkyl, Cycloalkyl und Aryl bedeuten und A⁻ für ein Anionäquivalent steht.

Vorzugsweise stehen R⁵ und R⁶ beide für C₁-C₄-Alkyl und insbesondere beide für Methyl.

Bevorzugt sind die Verbindungen der Formel I ausgewählt unter Verbindungen der Formel I.1 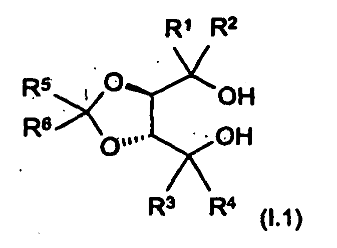 worin R¹, R², R³, R⁴, R⁵ und R⁶ die zuvor angegebenen Bedeutungen besitzen.

In dem erfindungsgemäßen Verfahren zur Auftrennung stereoisomerer Verbindungen, speziell Alkohole wird vorzugsweise ein Metallkomplex eingesetzt, der sich von einem Metall der I., II., III., IV. oder V. Hauptgruppe, der I, bis VIII. Nebengruppe, der Lanthanide oder der Actinide ableitet. Vorzugsweise ist das Metall ausgewählt unter B, Al, Ga, In, TI, Si, Ge, Sn, Pb, As, Sb, Bi, Ti, Zr, V, Cr, Mo, W, Mn, Fe, Ru, Os, Co, Rh, Ir, Ni, Pd, Pt, Cu, Ag, Au, Zn und Cd. Insbesondere handelt es sich bei dem Metall um Al, Ga, In.

Handelt es sich bei der aufzutrennenden Verbindung um einen stereoisomeren Monoalkohol, so ist das Metall vorzugsweise ein Metall, das zur Bildung einer stabilen dreiwertigen Oxidationsstufe befähigt ist. Diese sind vorzugsweise ausgewählt unter Al, Ga und In. Insbesondere handelt es sich bei dem Metall dann um Al.

Handelt es sich bei der aufzutrennenden Verbindung um eine stereoisomere Verbindung mit zwei funktionellen Gruppen, die ausgewählt sind unter Alkohol- und/oder Aminogruppen, eignen sich als Metall bevorzugt Metalle, die zur Bildung einer stabilen vierwertigen Oxidationsstufe befähigt sind. Dazu zählen insbesondere Ti, Zr, Ge, Sn. Insbesondere handelt es sich bei dem Metall dann um Titan.

Bei dieser Ausführungsform sind die aufzutrennenden Verbindungen dann insbesondere ausgewählt unter Diolen, Aminoalkoholen und Diaminen. Diese weisen vorzugsweise zwei Hydroxylgruppen oder zwei Aminogruppen oder eine Hydroxylgruppe und eine Aminogruppe jeweils in α- und β-Stellung zueinander auf. Geeignete Verbindungen sind die im Folgenden genannten.

Zusätzlich zu den zuvor beschriebenen Liganden der Formel I können die Diskriminatoren noch wenigstens einen weiteren Liganden aufweisen, der vorzugsweise ausgewählt ist unter Alkoholen, Thiolen, Aminen, Halogeniden, Alkanen, Carboxylaten, Acetylacetonat, Aryl- oder Alkylsulfonaten, Hydrid, N-haltigen Heterocyclen, Aromaten und Heteroaromaten.

Vorzugsweise ist der als chiraler Diskriminator eingesetzte Metallkomplex ausgewählt unter Verbindungen der allgemeinen Formel I.a 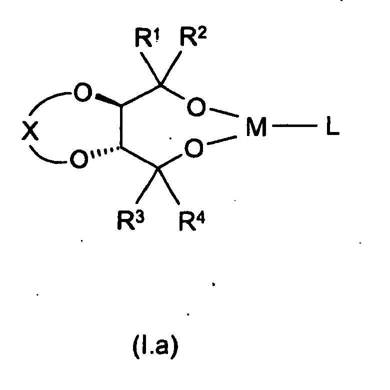 worin
- R¹, R², R³, R⁴ und X: die zuvor angegebenen Bedeutungen besitzen,
- M: für ein Metall, vorzugsweise Al, steht, und
- L: ausgewählt ist unter Alkyloxy, Cycloalkyloxy, Aryloxy, Arylalkyloxy, Alkylthio, Cycloalkylthio, Arylthio, Arylalkylthio, N,N-Dialkylamino, N,N-Dicycloalkylamino, N,N-Diarylamino, N,N-Diarylalkylamino, N-Aryl-N-alkylamino, N-Aryl-N-arylalkylamino, Alkyl, Cycloalkyl, Aryl oder Arylalkyl.

Bevorzugt leitet sich L von den zur Auftrennung eingesetzten stereoisomeren Alkoholen ab.

In einer alternativen bevorzugten Ausführungsform ist L ausgewählt unter gegebenenfalls substituierten aus Phenolen. Substituierte Phenole können vorzugsweise 1, 2, 3 oder 4 Substituenten aufweisen, die ausgewählt sind unter den eingangs für "Aryl" genannten Substituenten. Bevorzugt sind dabei Phenole mit mindestens einem Substituenten in der 2-Position. Vorzugsweise sind die Substituenten der Phenole ausgewählt unter C₁-C₄-Alkyl, besonders bevorzugt Methyl, Ethyl, n-Propyl, Isopropyl, n-Butyl oder tert.-Butyl, C₁-C₄-Alkoxy, bevorzugt Methoxy, Ethoxy, n-Propoxy, Isopropoxy, n-Butoxy, tert.-Butoxy, Halogen, bevorzugt Fluor oder Chlor, Trifluormethyl, Methoxycarbonyl oder Ethoxycarbonyl. Des Weiteren bevorzugt sind unsubstituierte Phenole.

In einer besonders bevorzugten Ausführungsform ist L ausgewählt aus Alkoholen oder Phenolen, die einen höheren Siedepunkt als das zu trennende Racemat haben.

Bevorzugt wird zur Herstellung des Metallkomplexes ein Aluminiumtrialkoholat, wie Aluminiumethylat (Aluminiumethoxid, Aluminiumtriethylat), Aluminium-n-propylat, Aluminiumisopropylat, Aluminium-n-butylat, Aluminium-sec.-butylat, Aluminium-tert.-butylat, Aluminiumphenolat, etc. eingesetzt.

Vorzugsweise ist der als chiraler Diskriminator eingesetzte Metallkomplex ausgewählt unter Verbindungen der allgemeinen Formel I.b 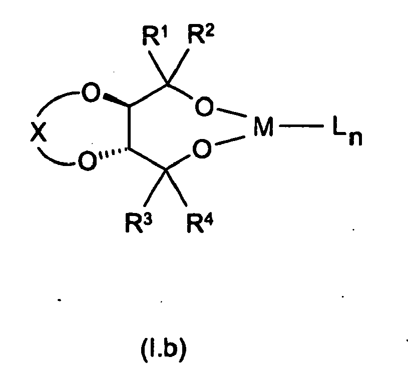 worin
n = in Abhängigkeit von der Wertigkeit des Metalls M für 1 oder 2 steht,
R¹, R², R³, R⁴ und X die zuvor angegebenen Bedeutungen besitzen,
M für ein Metall, vorzugsweise Al oder Ti steht, und
- L: ausgewählt ist unter Alkyloxy, Cycloalkyloxy, Aryloxy, Arylalkyloxy, Alkylthio, Cycloalkylthio, Arylthio, Arylalkylthio, N,N-Dialkylamino, N,N-Dicycloalkylamino, N,N-Diarylamino, N,N-Diarylalkylamino, N-Aryl-N-alkylamino, N-Aryl-N-arylalkylamino, Alkyl, Cycloalkyl, Aryl oder Arylalkyl.

Bevorzugt leitet sich L von den zur Auftrennung eingesetzten stereoisomeren Alkoholen ab.

In einer alternativen bevorzugten Ausführungsform ist L ausgewählt unter gegebenenfalls substituierten Phenolen. Substituierte Phenole können vorzugsweise 1, 2, 3 oder 4 Substituenten aufweisen, die ausgewählt sind unter den eingangs für "Aryl" genannten Substituenten. Bevorzugt sind dabei Phenole mit mindestens einem Substituenten in der 2-Position. Vorzugsweise sind die Substituenten der Phenole ausgewählt unter C₁-C₄-Alkyl, besonders bevorzugt Methyl, Ethyl, n-Propyl, Isopropyl, n-Butyl oder tert.-Butyl, C₁-C₄-Alkoxy, bevorzugt Methoxy, Ethoxy, n-Propoxy, Isopropoxy, n-Butoxy, tert.-Butoxy, Halogen, bevorzugt Fluor oder Chlor, Trifluormethyl, Methoxycarbonyl oder Ethoxycarbonyl. Des Weiteren bevorzugt sind unsubstituierte Phenole.

In einer besonders bevorzugten Ausführungsform ist L ausgewählt aus Alkoholen oder Phenolen, die einen höheren Siedepunkt als das zu trennende Racemat haben.

Bevorzugt wird zur Herstellung des Metallkomplexes ein Aluminiumtrialkoholat, wie Aluminiumethylat (Aluminiumethoxid, Aluminiumtriethylat), Aluminium-n-propylat, Aluminiumisopropylat, Aluminium-n-butylat, Aluminium-sec.-butylat, Aluminium-tert.-butylat, Aluminiumphenolat, etc. oder ein Titantetraalkoholat, wie Titanethylat, Titan-n-propylat, Titanisopropylat, Titan-n-butylat, Titan-sec-butylat, Titan-tert.-butylat, Titanphenolat, etc. eingesetzt.

Vorzugsweise ist der als chiraler Diskriminator eingesetzte Metallkomplex ausgewählt unter Verbindungen der allgemeinen Formel I.b1 oder I.b2 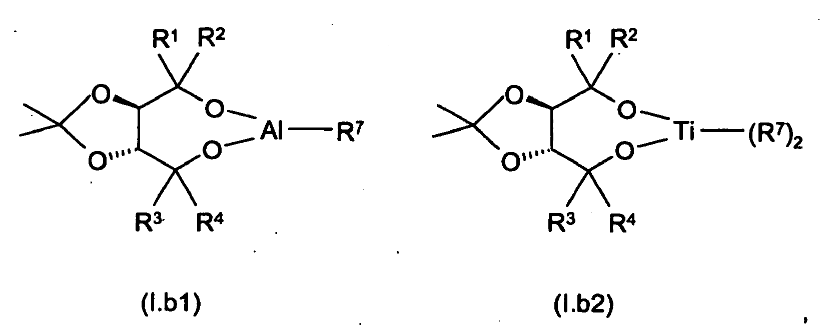 worin
R¹, R², R³ und R⁴ die zuvor angegebenen Bedeutungen besitzen und
R⁷ ausgewählt ist unter C₁-C₂₅-Alkyloxy, vorzugsweise C₁-C₈-Alkoxy, und Phenoxy.

Im Folgenden werden beispielhaft einige geeignete Diskriminatoren gezeigt

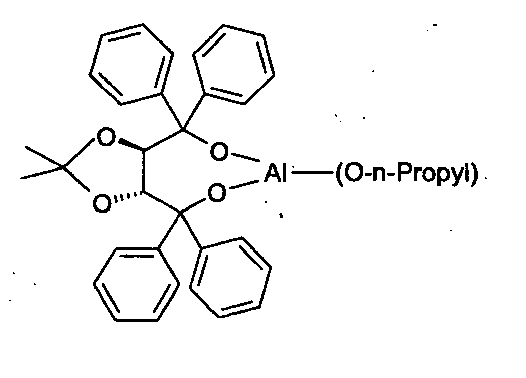

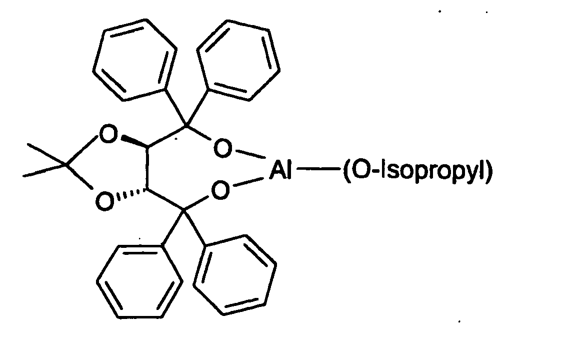

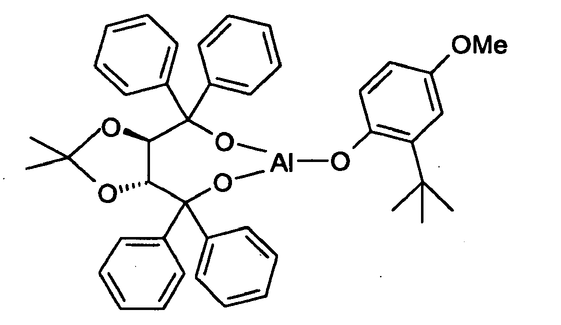

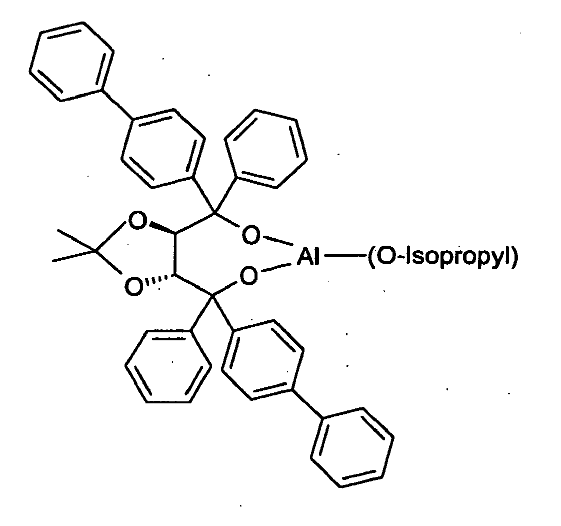

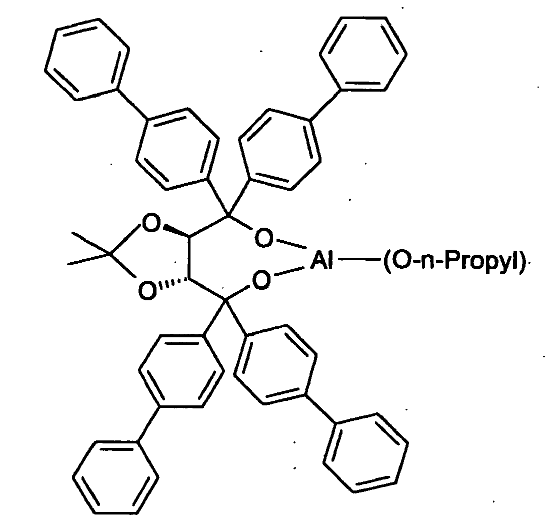

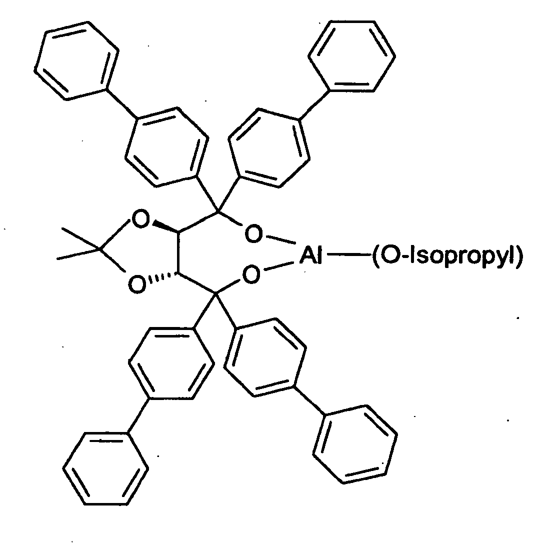

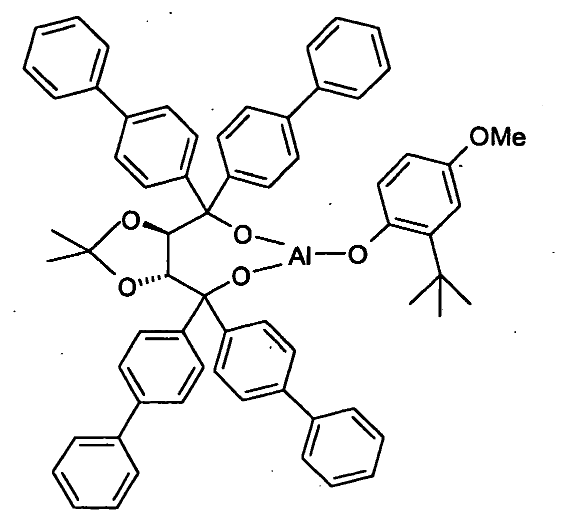

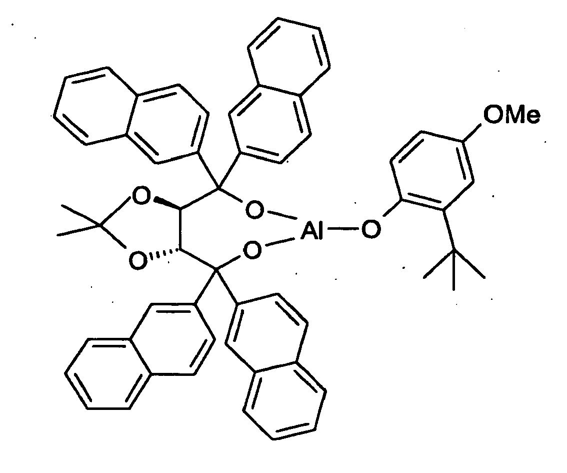

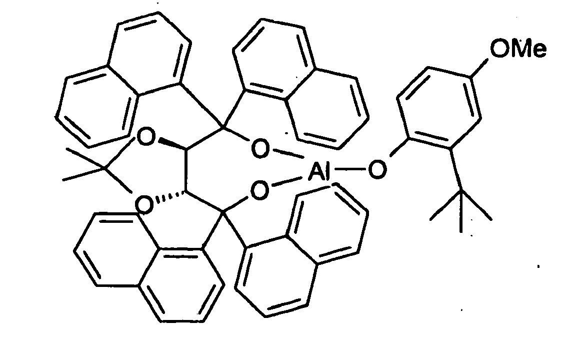

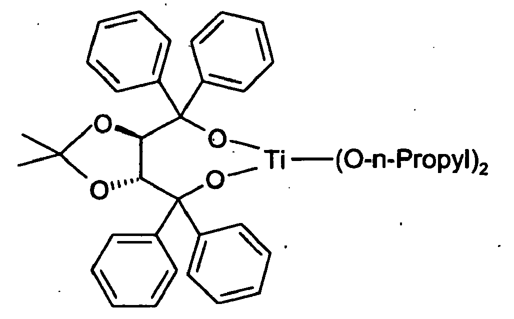

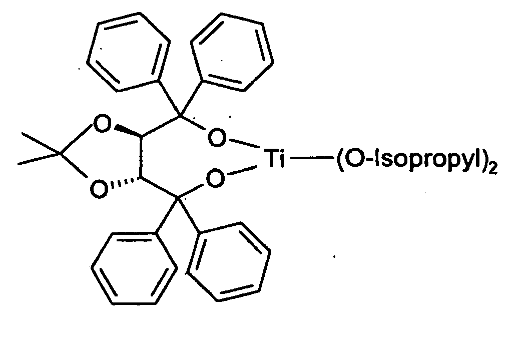

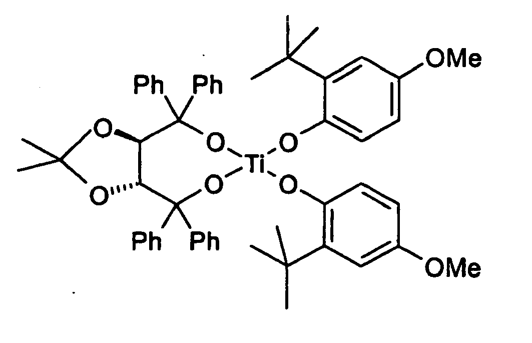

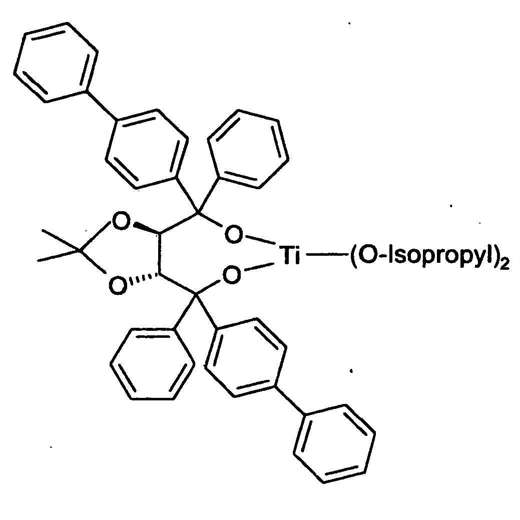

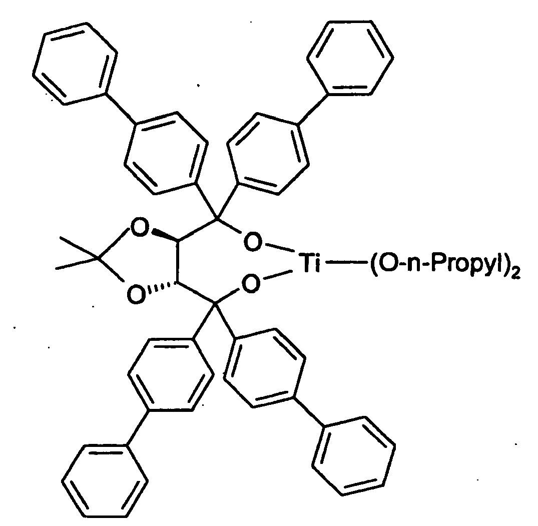

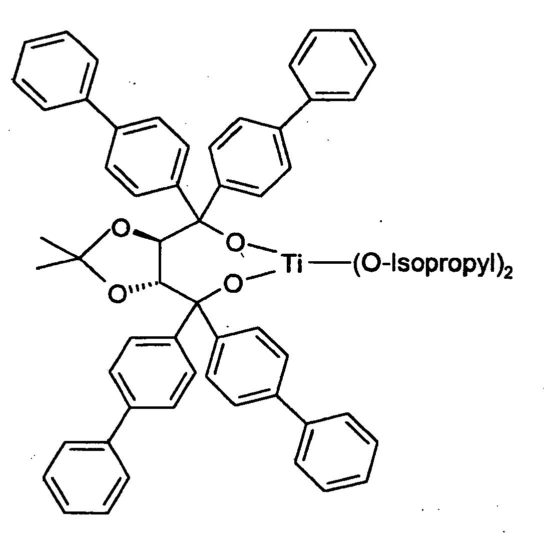

Die erfindungsgemäß eingesetzten chiralen Diskriminatoren führen zu einer Siedepunktsdifferenz der stereoisomeren Verbindungen, speziell Alkohole, die wenigstens eine Alkohol- und/oder Aminogruppe aufweisen (z. B. sofern es sich um enantiomere Verbindungen handelt) oder zu einer Vergrößerung einer bereits bestehenden Siedepunktsdifferenz der stereoisomeren Verbindungen, speziell Alkohole, die wenigstens eine Alkohol- und/oder Aminogruppe aufweisen (z. B. sofern es sich um diastereomere Verbindungen handelt).

Bevorzugt enthält das zur Auftrennung eingesetzte Gemisch zwei stereoisomere Verbindungen, speziell Alkohole. Bevorzugt handelt es sich bei den stereoisomeren Verbindungen um Enantiomere eines Alkohols.

Besonders bevorzugt handelt es sich bei den stereoisomeren Verbindungen um Alkohole der allgemeinen Formel (III) 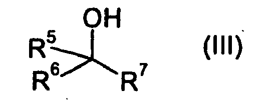 wobei
- R⁵ und R⁶: unabhängig voneinander ausgewählt sind unter Alkyl, Cycloalkyl, Heterocycloalkyl, Aryl und Hetaryl, die jeweils gegebenenfalls substituiert sein können;
- R⁷: für Wasserstoff steht oder eine der für R⁵ und R⁶ gegebenen Bedeutungen besitzt; und
wobei die Substituenten R⁵, R⁶ und R⁷ jeweils unterschiedlich voneinander sind.

Insbesondere handelt es sich bei den stereoisomeren Verbindungen der allgemeinen Formel (III) um die Enantiomere dieser Verbindungen.

Geeignete Alkyl-, Cycloalkyl-, Heterocycloalkyl-, Aryl- und Hetaryl-Substituenten sind die eingangs erwähnten.

Alkyl steht vorzugsweise für C₁-C₈-Alkyl. Cycloalkyl steht vorzugsweise für C₃-C₈-Cycloalkyl. Aryl steht vorzugsweise für Phenyl oder Naphthyl, speziell für Phenyl.

Bevorzugt stehen die Substituenten R⁵ und R⁶ unabhängig voneinander für unsubstituiertes oder gegebenenfalls durch Halogen substituiertes C₁-C₄-Alkyl, speziell für gegebenenfalls substituiertes Benzyl, oder für gegebenenfalls durch Halogen, Cyano, Nitro, C₁-C₄-Alkyl oder C₁-C₄-Alkoxy substituiertes Aryl, insbesondere für gegebenenfalls substituiertes Phenyl oder Naphthyl. R⁷ steht in dieser Ausführungsform bevorzugt für Wasserstoff.

Beispielhaft für in dem erfindungsgemäßen Verfahren einsetzbaren Monoalkoholen seien (RS)1-Phenylethanol, (RS)-2-Butinol, (RS)-2-Pentinol, (RS)1-Phenylpropanol, (RS)1-(4-Chlorphenyl)ethanol, (RS)1-(4-Chlorphenyl)propanol, (RS)2-Chlor-1-phenylethanol, (RS)3-Chlor-1-Phenylpropanol, (RS)2-Chlor-1-(4-Chlorphenyl)ethanol, (RS)3-Chlor-1-(4-Chlorphenyl)propanol, (RS)2-Chlor-1-(3-Chlorphenyl)ethanol, (RS)3-Chlor-1-(3-Chlorphenyl)propanol, (RS)2-Chlor-1-(2-Chlorphenyl)ethanol, (RS)3-Chlor-1-(2-Chlorphenyl)propanol, (RS)1-(4-Nitrophenyl)ethanol, (RS)1-(4-Nitrophenyl)propanol, (RS) 1-Naphthylethanol, (RS)1-Naphthylpropanol, (RS)1-(6-Methoxynaphthyl)ethanol, (RS)1-(6-Methoxynaphthyl)propanol, (RS)2-Chlor-1-naphthylethanol, (RS)3-Chlor-1-naphthylpropanol, (RS)2-Chlor-1-(6-methoxynaphthyl)ethanol, (RS)3-Chlor-1-(6-methoxynaphthyl)propanol, (RS)1-(4-Methylphenyl)ethanol, (RS)1-(4-Methylphenyl)propanol, (RS)2-Chlor-1-(4-Methylphenyl)ethanol, (RS)3-Chlor-1-(4-Methylphenyl)propanol, (RS)1-(4-Ethylphenyl)ethanol, (RS)1-(4-Ethylphenyl)propanol, (RS)2-Chlor-1-(4-ethylphenyl)ethanol, (RS)3-Chlor-1-(4-ethylphenyl)propanol, (RS)1-(4-Methoxyphenyl)ethanol, (RS)1-(4-Methoxyphenyl)propanol, (RS)2-Chlor-1-(4-methoxyphenyl)ethanol, (RS)3-Chlor-1-(4-methoxyphenyl)propanol, (RS)1-(2-Methylphenyl)ethanol, (RS)1-(2-Methylphenyl)propanol, (RS)2-Chlor-1-(2-Methylphenyl)ethanol, (RS)3-Chlor-1-(2-Methylphenyl)propanol, (RS)1-(2-Ethylphenyl)ethanol, (RS)1-(2-Ethylphenyl)propanol, 2-Chlor-1-(2-ethylphenyl)ethanol, (RS)3-Chlor-1-(2-ethylphenyl)propanol, (RS)1-(2-Methoxyphenyl)ethanol, (RS)1-(2-Methoxyphenyl)propanol, 2-Chlor-1-(2-methoxyphenyl)ethanol, (RS)3-Chlor-1-(2-methoxyphenyl)propanol, (RS)1-(3-Methylphenyl)ethanol, (RS)1-(3-Methylphenyl)propanol, (RS)2-Chlor-1-(3-Methylphenyl)ethanol, (RS)3-Chlor-1-(3-Methylphenyl)propanol, (RS)1-(3-Ethylphenyl)ethanol, (RS)1-(3-Ethylphenyl)propanol, (RS)2-Chlor-1-(3-ethylphenyl)ethanol, (RS)3-Chlor-1-(3-ethylphenyl)propanol, (RS)1-(3-Methoxyphenyl)ethanol, (RS)1-(3-Methoxyphenyl)propanol, (RS)2-Chlor-1-(3-methoxyphenyl)ethanol, (RS)3-Chlor-1-(3-methoxyphenyl)propanol und (RS)1-(1,3)-Benzodioxolethanol genannt.

In einer weiteren bevorzugten Form enthält das zur Auftrennung eingesetzte Gemisch stereoisomere Verbindungen, insbesondere ausgewählt unter Diolen, Aminoalkoholen und Diaminen. Diese weisen vorzugsweise zwei Hydroxylgruppen oder zwei Aminogruppen oder eine Hydroxylgruppe und eine Aminogruppe jeweils in α- und β-Stellung zueinander auf. Bevorzugt handelt es sich bei den stereoisomeren Verbindungen um Enantiomere oder Diastereomere.

Besonders bevorzugt handelt es sich bei den stereoisomeren Verbindungen um Verbindungen der allgemeinen Formel (IV) 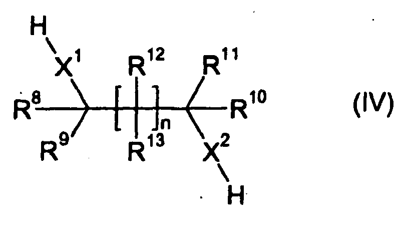 wobei
- X¹ und X²: unabhängig voneinander für O oder NR¹⁴ stehen, wobei R¹⁴ ausgewählt ist unter Wasserstoff, Alkyl, Cycloalkyl und Aryl, die jeweils gegebenenfalls substituiert sein können;
- n: für 0 oder 1 steht; und
wobei mit der Maßgabe, dass R⁸ und R⁹ voneinander unterschiedlich sind und/oder R¹⁰ und R¹¹ voneinander unterschiedlich sind und/oder R¹² und R¹³ wenn vorhanden voneinander unterschiedlich sind,
- R⁸, R⁹, R¹⁰, R¹¹: unabhängig voneinander ausgewählt sind unter Wasserstoff, Alkyl, Cycloalkyl, Heterocycloalkyl, Aryl und Hetaryl, die jeweils gegebenenfalls substituiert sein können; oder
R⁸ zusammen mit R¹⁰ und den Kohlenstoffatomen, über die diese miteinander verbunden sind, einen 3- bis 8-gliedrigen, gesättigten oder ungesättigten, nicht-aromatischen Carbocyclus bilden, der gegebenenfalls substituiert ist und weitere anellierte gesättigte, ungesättigte oder aromatische Carbocyclen oder Heterocyclen umfassen kann; oder
wenn X¹ und/oder X² für NR¹⁴ steht, R⁸ zusammen mit R¹⁴ und/oder R¹⁰ zusammen mit R¹⁴ und jeweils zusammen mit den Atomen, über die diese miteinander verbunden sind, einen gegebenenfalls substituierten, stickstoffhaltigen Ring bilden können;
- R¹² und R¹³: unabhängig voneinander eine der für R⁸, R⁹, R¹⁰, R¹¹ gegebenen Bedeutungen besitzt; oder
R¹² zusammen mit R⁸ und den Kohlenstoffatomen, über die diese miteinander verbunden sind, einen 3- bis 8-gliedrigen, gesättigten oder teilweise ungesättigten Carbocyclus bilden, der gegebenenfalls substituiert ist und weitere anellierte gesättigte, teilweise ungesättigte oder aromatische Carbocyclen oder Heterocyclen umfassen kann; oder
wenn X¹ und/oder X² für NR¹⁴ steht, R¹² zusammen mit R¹⁴ und/oder R¹³ zusammen mit R¹⁴ und jeweils zusammen mit den Atomen, über die diese miteinander verbunden sind, einen gegebenenfalls substituierten, stickstoffhaltigen Ring bilden können.

Geeignete Alkyl-, Cycloalkyl-, Heterocycloalkyl-, Aryl- und Hetaryl-Substituenten sind die eingangs erwähnten.

Alkyl steht vorzugsweise für C₁-C₈-Alkyl. Cycloalkyl steht vorzugsweise für C₃-C₈-Cycloalkyl. Aryl steht vorzugsweise für Phenyl oder Naphthyl, speziell für Phenyl.

Insbesondere bevorzugt sind Verbindungen der Formel (IV) worin n für 0 steht.

Weiterhin stehen R¹² und R¹³ unabhängig voneinander bevorzugt für Wasserstoff oder C₁-C₆-Alkyl, wobei die Radikale R¹² und R¹³ gleich oder voneinander verschieden sein können.

Beispielhaft für in dem erfindungsgemäßen Verfahren einsetzbare stereoisomerer Verbindungen mit zwei zueinander α und β-ständigen funktionellen Gruppen, die Alkohol und/oder Amin sein können, seien racemische Mischungen von 1,2-Propandiol, 3-Chlor-1,2-propandiol, 3-Brom-1,2-propandiol, 1,2-Butandiol, 2,3-Butandiol, 1,4-Dichlor-2,3-Butandiol, 2-Methyl-2,3-butandiol, 1,2-Pentandiol, 1,2-Cyclopentandiol, trans-1,2-Cyclopentandiol, 1,2-Hexandiol, 1,2-Cyclohexandiol, trans-1,2-Cyclohexandiol,
3,3-Dimethyl-1,2-butandiol, 1,2-Heptandiol, 2,3-Heptandiol, 1,2-Cycloheptandiol, trans-1,2-Cycloheptandiol, 1,2-Octandiol,
7-Octen-1,2-diol, 1-Phenyl-1,2-ethandiol, 1-Phenyl-1,2-propandiol,
2-Phenyl-1,2-propandiol, 1-Isopropenyl-4-methyl-1,2-cyclohexandiol,
1,2,3,4-Tetrahydro-2,3-naphtalendiol, 2-Amino-3-hydroxybutanol (Threoninol), 1-Amino-2-propanol, 2-Amino-1-propanol, 1-Amino-2-Butanol, 2-Amino-1-Butanol, 2-Amino-2-methyl-1-propanol, 2-Amino-3-Methyl-1-butanol (Valinol), 2-Amino-1-pentanol, 2-Amino-4-methylmercapto-1-butanol (Methioninol), 2-Amino-3-methyl-1-pentanol (Isoleucinol), 2-Amino-4-methyl-1-pentanol (Leucinol), 2-Amino-3,3-dimethyl-1-butanol (tert-Leucinol), 2-Amino-1-hexanol, 2-Aminocyclohexanol, 2-Ethylamino-1-butanol, 2-Amino-1-phenylethanol, 2-Amino-2-phenylethanol (α-Phenylglycinol), 2-Amino-3-phenyl-1-propanol (Phenylalanol), 2-Amino-1-phenyl-1-propanol, 2-Amino-3-(p-chlorophenyl)-1-propanol, 2-Benzylamino-1-propanol, 2-Amino-2-Methyl-3-phenyl-1-propanol, 1-Amino-2-indanol, 2-Amino-1-indanol,
3-Amino-1,2,3,4-tetrahydro-2-naphtol, 2-Amino-3-(a-imidazolyl)propanol (Histidinol), 2-(Hydroxymethyl)pyrrolidin (Prolinol),1,2-Propylendiamin, 2-Methyl-1,2-propandiamin, 1,2-Cyclopentandiamin, trans-1,2-Cyclopentandiamin,1,2-Cyclohexandiamin, trans-1,2-Cyclohexandiamin, 1,1-Di-(4-anisyl)-2-isopropyl-ethylendiamin (Daipen),1,3-Butandiol, 1,3-Pentandiol, 2,2,4-Trimethyl-1,3-pentandiol, 1,3-Cyclohexandiol, 2-Methyl-2,4-pentandiol, 2,4-Pentandiol, 3-Methyl-2,4-pentandiol, 1,3-Cyclohexandiamin und 3-Amino-2-methyl-1-phenyl-1-propanol genannt.

Zur Durchführung des erfindungsgemäßen Verfahrens werden die aufzutrennenden stereoisomeren mit einem chiralen Diskriminator, wie zuvor beschrieben in Kontakt gebracht und gleichzeitig und/oder anschließend einer destillativen Trennung unterzogen.

Die durch die erfindungsgemäß eingesetzten Diskriminatoren induzierte Siedepunktsdifferenz zwischen den aufzutrennenden stereoisomeren Verbindungen ermöglicht eine destillative Auftrennung. Diese kann prinzipiell nach üblichen, dem Fachmann bekannten Destillationsverfahren erfolgen. Geeignete Vorrichtungen zur destillativen Aufarbeitung umfassen Destillationskolonnen, wie Bodenkolonnen, die mit Glocken, Siebplatten, Siebböden, Ventilen, Seitenabzügen, etc. ausgerüstet sein können, Verdampfer, wie Dünnschichtverdampfer, Sambay-Verdampfer etc und Kombinationen davon.

Selbstverständlich kann zum in Kontakt bringen des Diskriminators mit den aufzutrennenden stereoisomeren Verbindungen und/oder zur Trennung auch ein inertes Lösungsmittel eingesetzt werden. Bevorzugt handelt es sich dabei um ein Lösungsmittel, das es ermöglicht die Destillation, wie im Folgenden näher beschrieben, als Extraktivdestillation durchzuführen. In der Regel geeignete Lösungsmittel sind z. B. Aromaten, wie Toluol und Xylole, Kohlenwasserstoffe oder Gemische von Kohlenwasserstoffen. Weiterhin geeignet sind halogenierte, insbesondere chlorierte Kohlenwasserstoffe oder Aromaten, wie 2-Chlornaphthalin. Weitere geeignete Lösungsmittel sind Ester aliphatischer oder aromatischer Carbonsäuren mit Alkanolen, beispielsweise Texanol® (2,2,4-Trimethyl-1,3-pentandiolmonobutyrat) oder Palatinole. Geeignete Lösungsmittel sind weiterhin Ether, vorzugsweise Diarylether, wie Diphenylether, und Dialkylether, wie Di-n-butylether oder Polyether, beispielsweise Lutrole® (Polyethylenglykol). Geeignete Lösungsmittel sind weiterhin Carbonsäureamide, wie N,N-Dialkylcarbonsäureamide, z. B. Dimethylformamid und Dimethylacetamid, sowie Dimethylacetanilid oder Carbonsäurelactame, wie N-Alkylcarbonsäurelactame, z. B. N-Methylpyrrolidon. Weiterhin geeignet sind schwefelhaltige Lösungsmittel, wie Dimethylsulfoxid oder Sulfolan.

Auch können technische Weißöle, Thermoöle und Wärmeträgerflüssigkeiten, wie Marlotherme®, Dowtherme®, Therminol® als Lösungsmittel verwendet werden. Auch können Ketone, wie Cyclohexanon, etc., eingesetzt werden. Ferner können als Lösungsmittel auch so genannte "ionische Flüssigkeiten" verwendet werden. Hierbei handelt es sich um flüssige Salze, beispielsweise um N,N'-Dialkylimidazoliumsalze wie die N-Butyl-N'-methylimidazoliumsalze, Tetraalkylammoniumsalze wie die Tetra-n-butylammoniumsalze, N-Alkylpyridiniumsalze wie die n-Butylpyridiniumsalze, Tetraalkylphosphoniumsalze wie die Trishexyl(tetradecyl)phosphoniumsalze, z. B. die Tetrafluoroborate, Acetate, Tetrachloroaluminate, Hexafluorophosphate, Chloride und Tosylate. Als weiteres geeignetes Lösungsmittel kann das zu trennende Racemat selbst dienen.

In einer bevorzugten Ausführung des efindungsgemäßen Verfahrens erfolgt die Trennung durch Extraktivdestillation. Die Extraktivdestillation ist ein prinzipiell bekanntes Verfahren zur Auftrennung von Gemischen aus Komponenten, die sich nur geringfügig in ihren relativen Flüchtigkeiten unterscheiden oder die azeotrop sieden. Die Extraktivdestillation wird unter Zugabe eines zusätzlichen (selektiven) Lösungsmittels, auch als Extraktionsmittel bezeichnet, durchgeführt, das gegenüber dem aufzutrennenden Gemisch bevorzugt höher siedet.

Vorzugsweise wird zur Extraktivdestillation ein zusätzliches Lösungsmittel eingesetzt, dessen Siedepunkt bei dem Druck, unter dem die Auftrennung erfolgt, um wenigstens 5 °C, besonders bevorzugt um wenigstens 10 °C, höher liegt, als der Siedepunkt des am höchsten siedenden Stereoisomers.

Für die Extraktivdestillation geeignete zusätzliche Lösungsmittel sind die zuvor genannten.

Bevorzugt wird ein zusätzliches Lösungsmittel eingesetzt, in dem der eingesetzte chirale Diskriminator unter den Auftrennungsbedingungen eine Löslichkeit von mindestens 20 g/l, insbesondere von mindestens 50 g/l, aufweist.

Das Verfahren kann bei Normaldruck, bei reduziertem oder bei erhöhtem Druck durchgeführt werden. Bevorzugt wird das Verfahren bei reduziertem Druck so ausgeübt, dass die Kopftemperatur der Kolonne weniger als 200 °C, bevorzugt weniger als 150 °C, besonders bevorzugt weniger als 100 °C beträgt.

Die erfindungsgemäße Destillation erfolgt üblicherweise bei einem Druck im Bereich von 0,01 bis 900 mbar, vorzugsweise 0,1 bis 500 mbar.

Die Extraktivdestillation in Gegenwart des Diskriminators kann mehrfach wiederholt werden. Die Anzahl der Wiederholungen richtet sich nach der gewünschten Reinheit der enantiomeren oder diastereomeren Produkte und kann vom Fachmann mit an sich bekannten Methoden bestimmt werden.

Die Erfindung wird anhand der folgenden, nicht einschränkenden Beispiele näher erläutert.

### Beispiele

### Beispiel 1:

### Trennung von racemisch.-1-Phenylethanol mit dem chiralen Selektor erzeugt aus (4R,5R)-bis(-Hydroxydiphenylmethyl)-2,2-dimethyl-1,3-dioxolan und Al(O-Isopropyl)₃

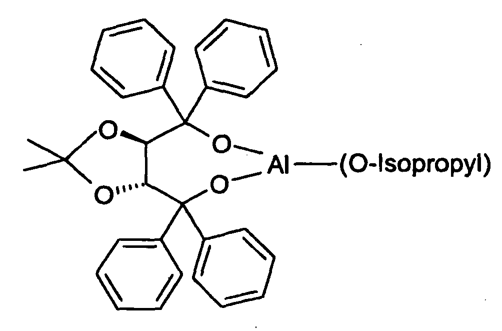

Zu 15 mmol (7,0 g) (4R,5R)-bis(Hydroxydiphenylmethyl)-2,2-dimethyl-1,3-dioxolan und 15 mmol (3,1 g) Aluminiumtriisopropoxid werden in einer Glovebox in 50 ml Diphenylether gegeben. Die entstandene Suspension wird bei 90 °C unter Schutzgas für 15 min gerührt. Im Anschluss werden 30 mmol (3,7 g) racemisch.-1-Phenylethanol zugegeben und für weitere 90 min gerührt und anschließend bei etwa 20 mbar das entstandene Isopropanol abdestilliert. Danach wird bei 1 mbar und 90 °C ca. 1 ml Phenylethanol abdestilliert. Untersuchung der Probe durch chirale GC liefert eine Zusammensetzung von 58 % (R)-1-Phenylethanol und 42 % (S)-1-Phenylethanol. Nach Hydrolyse des Destillationssumpfes wird das im Sumpf verbliebene 1-Phenylethanol durch chirale GC untersucht. Es wird eine Zusammensetzung von 46,6 % (R)-Phenylethanol und 53,4 % (S)-Phenylethanol gefunden.
GC: Säulenschaltung mit Vorsäule: 25 m * 0,25 mm Optioma-1 (Macherey & Nagel)
FD = 0,5 µm, und chirale Säule: 30 m * 0,25 mm BGB174S (BGB-Analytikvertrieb)
FD = 0,25 µm; Ofentemp.: 115 °C 12', 5°/*, 200 °C; Vorsäule: 1,4 bar H₂, chirale Säule: 1,1 bar H₂; Säulenschaltung: bei 1,9 min auf chirale Säule; RT R-1-Phenylethanol = 9,4 min; RT S-1-Phenylethanol = 9,8 min.

### Beispiel 2:

### Trennung von racemisch-1-Phenylethanol mit dem chiralen Selektor erzeugt aus (4R,5R)-bis-(Hydroxydi(4'-biphenylyl)methyl)-2,2-dimethyl-1,3-dioxolan und Al(O-Isopropyl)₃

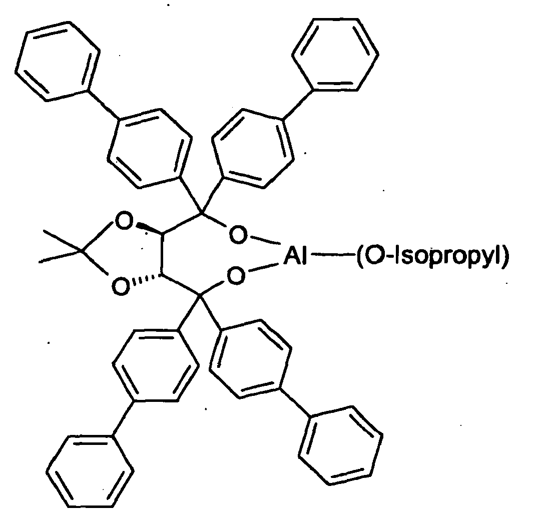

Zu 15 mmol (7,0 g) (4R,5R)-bis(Hydroxydi(4'-biphenylyl)methyl)-2,2-dimethyl-1,3-dioxolan und 15 mmol (3,1 g) Aluminiumtriisopropoxid werden in einer Glovebox in 50 ml Diphenylether gegeben. Die entstandene Suspension wird bei 90 °C unter Schutzgas für 15 min gerührt. Im Anschluss werden 30 mmol (3,7 g) racemisch-1-Phenylethanol zugegeben und ca. 1 ml Phenylethanol abdestilliert. Untersuchung der Probe durch chirale GC liefert eine Zusammensetzung von 53,4 % (R)-1-Phenylethanol und 46,6 % (S)-1-Phenylethanol.
GC: Säulenschaftung mit Vorsäule: 25 * 0,25 mm Optima-1 (Macherey & Nagel)
FD = 0,5 µm, und chirale Säule: 30 m * 0,25 mm BGB174S (BGB-Analytikvertrieb)
FD = 0,25 µm; Ofentemp.: 115 °C 12', 5°/*, 200 °C; Vorsäule: 1,4 bar H₂, chirale Säule: 1,1 bar H₂; Säulensclialtung: bei 1,9 min auf chirale Säule; RT R-1-Phenylethanol = 9,4 min; RT S-1-Phenylethanol = 9,8 min.

### Beispiel 3:

### Trennung von rac.-1,2-Propandiol mit dem chiralen Selektor erzeugt aus (4R,5R)-bis(hydroxydiphenylmethyl)-2,2-dimethyl-1,3-dioxolan und Ti(O-Isopropyl)₄

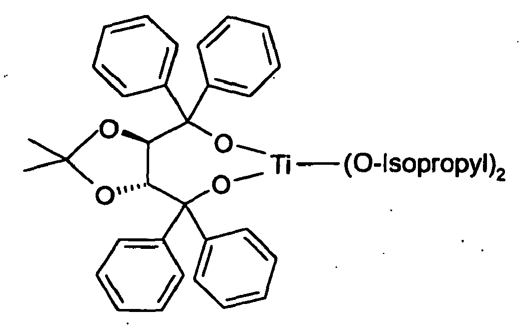

6,5 mmol (3,0 g) (4R,5R)-bis(Hydroxydiphenylmethyl)-2,2-dimethyl-1,3-dioxolan und 6,5 mmol (1,83 g) Titantetraisopropoxid werden in einer Glove-Box in 20 ml Diphenylether gegeben. Die entstandene gelbe Lösung wird bei 90 °C unter Schutzgas für 60 min gerührt. Im Anschluss werden 13 mmol (0,98 g) rac.-1,2-Propandiol zugegeben und für weitere 10 min gerührt und anschließend wird bei etwa 20 mbar das entstandene Isopropanol abdestilliert. Danach wird bei 1 mbar und 90 °C ca. 0,75 g eines Gemischs aus Diphenylether und 1,2-Propandiol abdestilliert. Der gaschromatographisch (Chirasil Dex CB, TFA-Derivatisierung) bestimmte Enantiomerenüberschuss für (S)-1,2-Propandiol beträgt 16 %.

### Beispiel 4:

### Trennung von rac.-1,2-Propandiol mit dem chiralen Selektor erzeugt aus (4R,5R)-bis(Hydroxydiphenylmethyl)-2,2-dimethyl-1,3-dioxolan und Ti(O-Isopropyl)₄

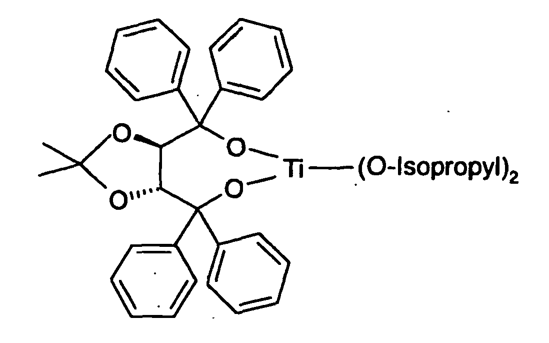

6,5 mmol (3,0 g) (4R,5R)-bis(Hydroxydiphenylmethyl)-2,2-dimethyl-1,3-dioxolan und 6,5 mmol (1,83 g) Titantetraisopropoxid werden in einer Glove-Box in 20 ml Diphenylether gegeben: Die entstandene gelbe Lösung wird bei 90 °C unter Schutzgas für 60 min gerührt. Im Anschluss werden 26 mmol (1,96 g) rac.-1,2-Propandiol zugegeben und für weitere 10 min gerührt und anschließend wird bei etwa 20 mbar das entstandene Isopropanol abdestilliert. Danach wird bei 1 mbar und 90 °C ca. 0,75 g eines Gemischs aus Diphenylether und 1,2-Propandiol abdestilliert. Der gaschromatographisch (Chirasil Dex CB, TFA-Derivatisierung) bestimmte Enantiomerenüberschuss für (S)-1,2-Propandiol beträgt 7,4 %.

### Beispiel 5:

### Trennung von rac.-2-Amino-1-propanol mit dem chiralen Selektor erzeugt aus (4R,5R)-bis(Hydroxydiphenylmethyl)-2,2-dimethyl-1,3-dioxolan und Ti(O-Isopropyl)₄

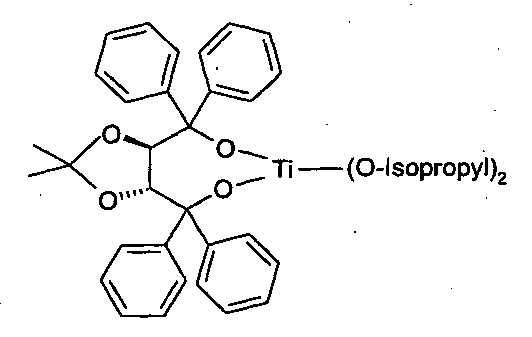

6,5 mmol (3,0 g) (4R,5R)-bis(Hydroxydiphenylmethyl)-2,2-dimethyl-1,3-dioxolan und 6,5 mmol (1,83 g) Titantetraisopropoxid werden in einer Glove-Box in 20 ml Diphenylether gegeben. Die entstandene gelbe Lösung wird bei 90 °C unter Schutzgas für 60 min gerührt. Im Anschluss werden 13 mmol (0,97 g) rac.-2-Amino-1-propanol zugegeben und für weitere 10 min gerührt und anschließend wird bei etwa 20 mbar das entstandene Isopropanol abdestilliert. Danach wird bei 1 mbar und 90 °C ca. 0,75 g eines Gemischs aus Diphenylether und 2-Amino-1-propanol abdestilliert. Der gaschromatographisch (Chirasil Dex CB, TFA-Derivatisierung) bestimmte Enantiomerenüber schuss für (S)-2-Amino-1-propanoi beträgt 6,3 %.

### Beispiel 6:

### Trennung von rac.-1-Phenylethanol mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor : Alkohol, 0,5 : 1)

6,4 mmol (3,0 g) (4R,5R)-bis(Hydroxydiphenylmethyl)-2,2-dimethyl-1,3-dioxolan und 6,4 mmol (1,31 g) Aluminiumtriisopropoxid werden in einer Glovebox in 20 ml Diphenylether gegeben. Die entstandene Suspension wird bei 90 °C unter Schutzgas für 60 min gerührt. Im Anschluss werden 12,8 mmol (1,56 g) racemisches 1-Phenylethanol zugegeben und für weitere 10 min gerührt. Anschließend wird bei etwa 20 mbar das entstandene Isopropanol abdestilliert. Danach werden bei 1 mbar und 90 °C 1,2 g eines Gemischs aus (R)-1-Phenylethanol und (S)-1-Phenylethanol als Destillat erhalten. Der durch chirale Gaschromatographie (BGB174S, BGB-Analytikvertrieb) bestimmte Enantiomerenüberschuss an (R)-1-Phenylethanol betrug 12,0 %.

### Beispiel 7:

### Trennung von rac.-1-Phenylethanol mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor : Alkohol, 2 : 1)

9,9 mmol (4,6 g) (4R,5R)-bis(Hydroxydiphenylmethyl)-2,2-dimethyl-1,3-dioxolan und 9,9 mmol (2,02 g) Aluminiumtriisopropoxid werden in einer Glovebox in 30 ml Diphenylether gegeben. Die entstandene Suspension wird bei 90°C unter Schutzgas für 60 min gerührt. Im Anschluss werden 4,95 mmol (0,60 g) racemisches 1-Phenylethanol zugegeben und für weitere 10 min gerührt und anschließend bei etwa 20 mbar das entstandene Isopropanol abdestilliert. Danach werden bei 1 mbar und 90 °C 1,0 g eines Gemischs aus (R)-1-Phenylethanof und (S)-1-Phenylethanol als Destillat erhalten. Der gaschromatographisch (BGB174S, BGB-Analytikvertrieb) bestimmte Enantiomerenüberschuss an (R)-1-Phenylethanol betrug 33,8 % ee.

### Beispiel 8:

### Trennung von rac.-1-Phenylethanol mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor: Alkohol, 3:1)

39,0 mmol (18,0 g) (4R,5R)-bis(Hydroxydiphenylmethyl)-2,2-dimethyl-1,3-dioxolan und 39,0 mmol (7,96 g) Aluminiumtriisopropoxid werden in einer Glovebox in 60 ml Diphenylether gegeben. Die entstandene Suspension wird bei 90 °C unter Schutzgas für 60 min gerührt. Im Anschluss werden 13 mmol (1,56 g) racemisches 1-Phenylethanol zugegeben und für weitere 10 min gerührt. Anschließend wird das entstandene Isopropanol bei etwa 20 mbar abdestilliert. Danach werden bei 1 mbar und 90 °C 2,35 g eines Gemischs aus (R)-1-Phenylethanol und (S)-1-Phenylethanol als Destillat erhalten. Der gaschromatographisch (BGB174S, BGB-Analytikvertrieb) bestimmte Enantiomerenüberschuss an (R)-1-Phenylethanol betrug 14,8 % ee.

### Beispiel 9:

### Trennung von rac.-1-Phenylethanol mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor: Alkohol, 4:1)

52,0 mmol (24,0 g) (4R,5R)-bis(Hydroxydiphenylmethyl)-2,2-dimethyl-1,3-dioxolan und 52,0 mmol (10,5 g) Aluminiumtriisopropoxid werden in einer Glovebox in 80 ml Diphenylether gegeben. Die entstandene Suspension wird bei 90 °C unter Schutzgas für 60 min gerührt. Im Anschluss werden 13 mmol (1,56 g) racemisches 1-Phenylethanol zugegeben und für weitere 10 min gerührt. Anschließend wird bei etwa 20 mbar das entstandene Isopropanol abdestilliert. Danach werden bei 1 mbar und 90 °C 3,7 g eines Gemischs aus (R)-1-Phenylethanol und (S)-1-Phenylethanol als Destillat erhalten. Der gaschromatographisch (BGB174S, BGB-Analytikvertrieb) bestimmte Enantiomerenüberschuss an (R)-1-Phenylethanol betrug 13,4 % ee.

### Beispiel 10:

### Trennung von rac.-4-Methylphenylethanol mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor: Alkohol, 1 : 1)

13,0 mmol (6,0 g) (4R,5R)-bis(Hydroxydiphenylmethyl)-2,2-dimethyl-1,3-dioxolan und 13,0 mmol (2,62 g) Aluminiumtriisopropoxid werden in einer Glovebox in 40 ml Diphenylether gegeben. Die entstandene Suspension wird bei 90°C unter Schutzgas für 60 min gerührt. Im Anschluss werden 13 mmol (1,75 g) racemisches 1-(4-Methylphenyl)ethanol zugegeben und für weitere 10 min gerührt. Anschließend wird bei etwa 20 mbar das entstandene Isopropanol abdestilliert. Danach wird bei 1 mbar und 90 °C 2,65 g Destillat erhalten. Untersuchung des Destillats durch chirale Gaschromatographie (BGB-Analytikvertrieb BGB-174S) ergab einen Enantiomerenüberschuss von 23,6 %.

### Beispiel 11:

### Trennung von rac.-4-Chlorphenylethanol mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor : Alkohol, 1 : 1)

13,0 mmol (6,0 g) (4R,5R)-bis(Hydroxydiphenylmethyl)-2,2-dimethyl-1,3-dioxolan und 13,0 mmol (2,62 g) Aluminiumtriisopropoxid werden in einer Glovebox in 40 ml Diphenylether gegeben. Die entstandene Suspension wird bei 90 °C unter Schutzgas für 60 min gerührt. Im Anschluss werden 13 mmol (2,02 g) racemisches 1-(4-Chlorphenyl)ethanol zugegeben und für weitere 10 min gerührt und anschließend bei etwa 20 mbar das entstandene Isopropanol abdestilliert. Danach wird bei 1 mbar und 90°C 1,4 g eines Gemischs der Enantiomere von 1-(4-Chlorphenyl)ethanol als Destillat erhalten. Untersuchung des Destillats durch chirale Gaschromatographie (BGB-Analytikvertrieb BGB-174S) ergab einen Enantiomerenüberschuss von 7,3 %.

### Beispiel 12:

### Trennung von rac.-4-Fluorphenylethanol mit einem chiralen Selektor erzeugt aus (4R,5R)-bis(Hydroxydiphenylmethyl)-2,2-dimethyl-1,3-dioxolan und AI(O-Isopropyl)₃ (Verhältnis von Selektor : Alkohol, 1:1)

Zu 13,0 mmol (6,0 g) (4R,5R)-bis(Hydroxydiphenylmethyl)-2,2-dimethyl-1,3-dioxolan und 13,0 mmol (2,62 g) Aluminiumtriisopropoxid werden in einer Glovebox in 40 ml Diphenylether gegeben. Die entstandene Suspension wird bei 90°C unter Schutzgas für 60 min gerührt. Im Anschluss werden 13 mmol (1,80 g) racemisches 1-(4-Fluorphenyl)ethanol zugegeben und für weitere 10 min gerührt und anschließend bei etwa 20 mbar das entstandene Isopropanol abdestilliert. Danach wird bei 1 mbar und 90 °C 1,25 g eines Gemischs der Enantiomere von 1-(4-Fluorphenyl)ethanol als Destillat erhalten. Untersuchung des Destillats durch chirale Gaschromatographie (BGB-Analytikvertrieb BGB-174S) ergab einen Enantiomerenüberschuss von 9,1 %.

### Beispiel 13:

### Trennung von rac.-3-(trifluormethyl)phenylethanol mit einem chiralen Selektor erzeugt aus (4R,5R)-bis(Hydroxydiphenylmethyl)-2,2-dimethyl-1,3-dioxolan und AI(O-Isopropyl)₃ (Verhältnis von Selektor : Alkohol, 1 : 1)

Zu 13,0 mmol (6,0 g) (4R,5R)-bis(Hydroxydiphenylmethyl)-2,2-dimethyl-1,3-dioxolan und 13,0 mmol (2,62 g) Aluminiumtriisopropoxid werden in einer Glovebox in 40 ml Diphenylether gegeben. Die entstandene Suspension wird bei 90 °C unter Schutzgas für 60 min gerührt. Im Anschluss werden 13 mmol (2,50 g) racemisches 1-(3-Trifluormethylphenyl)ethanol zugegeben und für weitere 10 min gerührt und anschließend bei etwa 20 mbar das entstandene Isopropanol abdestilliert. Danach wird bei 1 mbar und 90 °C 1,25 g eines Gemischs der Enantiomere von 1-(3-Trifluormethylphenyl)ethanol als Destillat erhalten. Untersuchung des Destillats durch chirale Gaschromatographie (BGB-Analytikvertrieb BGB-174S) ergab einen Enantiomerenüberschuss von 27,8 %.

### Beispiel 14:

### Optische Aufreinigung von 1-Phenylethanol (28% ee) mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor : Alkohol, 2:1)

Zu 26,0 mmol (12,0 g) (4R,5R)-bis(Hydroxydiphenylmethyl)-2,2-dimethyl-1,3-dioxolan und 26,0 mmol (5,25 g) Aluminiumtriisopropoxid werden in einer Glovebox in 60 ml Diphenylether gegeben. Die entstandene Suspension wird bei 90 °C unter Schutzgas für 60 min gerührt. Im Anschluss werden 13 mmol (1,56 g) Phenylethanol mit einem Enantiomerenüberschuss an (R)-1-Phenylethanol von 28 % ee zugegeben und für weitere 10 min gerührt. Anschließend wird bei etwa 20 mbar das entstandene Isopropanol abdestilliert. Danach wird bei 1 mbar und 90 °C 1,10 g eines Gemischs aus (R)-1-Phenylethanol und (S)-1-Phenylethanol als Destillat erhalten. Untersuchung des Destillats durch chirale Gaschromatographie (BGB-Analytikvertrieb BGB-174S) ergab einen Enantiomerenüberschuss an (R)-1-Phenylethanol von 49,1 %.

### Beispiel 15:

### Optische Aufreinigung von 1-Phenylethanol mit 47% ee mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor : Alkohol, 2 : 1)

Zu 26,0 mmol (12,0 g) (4R,5R)-bis(Hydroxydiphenylmethyl)-2,2-dimethyl-1,3-dioxolan und 26,0 mmol (5,25 g) Aluminiumtriisopropoxid werden in einer Glovebox in 60 ml Diphenylether gegeben. Die entstandene Suspension wird bei 90 °C unter Schutzgas für 60 min gerührt. Im Anschluss werden 13 mmol (1,56 g) Phenylethanol mit einem Enantiomerenüberschus an (R)-1-Phenylethanol von 47 % ee zugegeben und für weitere 10 min gerührt. Anschließend wird bei etwa 20 mbar das entstandene Isopropanol abdestilliert. Danach wird bei 1 mbar und 90 °C 1,10 g eines Gemischs aus (R)-1-Phenylethanol und (S)-1-Phenylethanol als Destillat erhalten. Untersuchung des Destillats durch chirale Gaschromatographie (BGB-Analytikvertrieb BGB-174S) lieferte einen Enantiomerenüberschuss an (R)-1-Phenylethanol von 59,0 %.

### Beispiel 16:

### Optische Aufreinigung von 1-Phenylethanol mit 66 % ee mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor : Alkohol, 2 : 1)

26,0 mmol (12,0 g) (4R,5R)-bis(Hydroxydiphenylmethyl)-2,2-dimethyl-1,3-dioxolan und 26,0 mmol (5,25 g) Aluminiumtriisopropoxid werden in einer Glovebox in 60 ml Diphenylether gegeben. Die entstandene Suspension wird bei 90 °C unter Schutzgas für 60 min gerührt. Im Anschluss werden 13 mmol (1,56 g) 1-Phenylethanol mit einem Enantiomerenüberschuss an (R)-1-Phenylethanol von 66 % ee zugegeben und für weitere 10 min gerührt. Anschließend wird bei etwa 20 mbar das entstandene Isopropanol abdestilliert. Danach wird bei 1 mbar und 90°C 1,10 g eines Gemischs aus (R)-1-Phenylethanol und (S)-1-Phenylethanol als Destillat erhalten. Untersuchung des Destillats durch chirale Gaschromatographie (BGB-Analytikvertrieb BGB-174S) ergab einen Enantiomerenüberschuss an (R)-1-Phenylethanol von 72,8 %.

### Beispiel 17:

### Optische Aufreinigung von Phenylethanol mit 76 % ee mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor : Alkohol, 2 : 1)

26,0 mmol (12,0 g) (4R,5R)-bis(Hydroxydiphenylmethyl)-2,2-dimethyl-1,3-dioxolan und 26,0 mmol (5,25 g) Aluminiumtriisopropoxid werden in einer Glovebox in 60 ml Diphenylether gegeben. Die entstandene Suspension wird bei 90 °C unter Schutzgas für 60 min gerührt. Im Anschluss werden 13 mmol (1,56 g) 1-Phenylethanol mit einem Enantiomerenüberschüss an (R)-1-Phenylethanol von 76 % ee zugegeben und für weitere 10 min gerührt. Anschließend wird bei etwa 20 mbar das entstandene Isopropanol abdestilliert. Danach wird bei 1 mbar und 90 °C 1,10 g eines Gemischs aus (R)-1-Phenylethanoi und (S)-1-Phenylethanol als Destillat erhalten. Untersuchung des Destillats durch chirale Gaschromatographie (BGB-Analytikvertrieb BGB-174S) ergab einen Enantiomerenüberschuss an (R)-1-Phenylethanol von 83,0 %.

### Beispiel 18:

### Optische Aufreinigung von Phenylethanol mit 82 % ee mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor : Alkohol, 2 : 1)

26,0 mmol (12,0 g) (4R,5R)-bis(Hydroxydiphenylmethyl)-2,2-dimethyl-1,3-dioxolan und 26,0 mmol (5,25 g) Aluminiumtriisopropoxid werden in einer Glovebox in 60 ml Diphenylether gegeben. Die entstandene Suspension wird bei 90 °C unter Schutzgas für 60 min gerührt. Im Anschluss werden 13 mmol (1,56 g) 1-Phenylethanol mit einem Enantiomerenüberschuss an (R)-1-Phenylethanol von 82 % ee zugegeben und für weitere 10 min gerührt. Anschließend wird bei etwa 20 mbar das entstandene Isopropanol abdestilliert. Danach wird bei 1 mbar und 90 °C 1,10 g eines Gemischs aus (R)-1-Phenylethanol und (S)-1-Phenylethanol als Destillat erhalten. Untersuchung des Destillats durch chirale Gaschromatographie (BGB-Analytikvertrieb BGB-174S) ergab einen Enantiomerenüberschuss an (R)-1-Phenylethanol von 86,3 %.

### Beispiel 19:

### Optische Aufreinigung von Phenylethanol mit 86 % ee mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor : Alkohol, 2 : 1)

26,0 mmol (12,0 g) (4R,5R)-bis(Hydroxydiphenylmethyl)-2,2-dimethyl-1,3-dioxolan und 26,0 mmol (5,25 g) Aluminiumtriisopropoxid werden in einer Glovebox in 60 ml Diphenylether gegeben. Die entstandene Suspension wird bei 90 °C unter Schutzgas für 60 min gerührt. Im Anschluss werden 13 mmol (1,56 g) 1-Phenylethanol mit einem Enantiomerenüberschuss an (R)-1-Phenylethanol von 86 % ee zugegeben und für weitere 10 min gerührt. Anschließend wird bei etwa 20 mbar das entstandene Isopropanol abdestilliert. Danach wird bei 1 mbar und 90 °C 1,10 g eines Gemischs aus (R)-1-Phenylethanol und (S)-1-Phenylethanol als Destillat erhalten. Untersuchung des Destillats durch chirale Gaschromatographie (BGB-Analytikvertrieb BGB-174S) ergab einen Enantiomerenüberschuss an (R)-1-Phenylethanol von 89,9 %.

### Beispiel 20:

### Optische Aufreinigung von Phenylethanol mit 91 % ee mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor : Alkohol, 2 : 1)

26,0 mmol (12,0 g) (4R,5R)-bis(Hydroxydiphenylmethyl)-2,2-dimethyl-1,3-dioxolan und 26,0 mmol (5,25 g) Aluminiumtriisopropoxid werden in einer Glovebox in 60 ml Diphenylether gegeben. Die entstandene Suspension wird bei 90 °C unter Schutzgas für 60 min gerührt. Im Anschluss werden 13 mmol (1,56 g) 1-Phenylethanol mit einem Enantiomerenüberschuss am (R)-1-Phenylethanol von 91 % ee zugegeben und für weitere 10 min gerührt. Anschließend wird bei etwa 20 mbar das entstandene Isopropanol abdestilliert. Danach wird bei 1 mbar und 90 °C 1,10 g eines Gemischs aus (R)-1-Phenylethanol und (S)-1-Phenylethanol als Destillat erhalten. Untersuchung des Destillats durch chirale Gaschromatographie (BGB-Analytikvertrieb BGB-174S) ergab einen Enantiomerenüberschuss an (R)-1-Phenylethanol von 92,4 %.

### Beispiel 21:

### Optische Aufreinigung von 1-Phenylethanol mit 92,5 % ee mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor : Alkohol, 2 : 1)

26,0 mmol (12,0 g) (4R,5R)-bis(Hydroxydiphenylmethyl)-2,2-dimethyl-1,3-dioxolan und 26,0 mmol (5,25 g) Aluminiumtriisopropoxid werden in einer Glovebox in 60 ml Diphenylether gegeben. Die entstandene Suspension wird bei 90 °C unter Schutzgas für 60 min gerührt. Im Anschluss werden 13 mmol (1,56 g) 1-Phenylethanol mit einem Enantiomerenüberschuss an (R)-1-Phenylethanol von 92,5 % ee zugegeben und für weitere 10 min gerührt. Anschließend wird bei etwa 20 mbar das entstandene Isopropanol abdestilliert. Danach wird bei 1 mbar und 90 °C 1,10 g eines Gemischs aus (R)-1-Phenylethanol und (S)-1-Phenylethanol als Destillat erhalten. Untersuchung des Destillats durch chirale Gaschromatographie (BGB-Analytikvertrieb BGB-174S) ergab einen Enantiomerenüberschuss an (R)-1-Phenytethanol von 94,7%.

### Beispiel 22:

### Optische Aufreinigung von Phenylethanol mit 94,2 % ee mit einem chiralen Selektor erzeugt aus (4R,5R)-bis(Hydroxydiphenylmethyl)-2,2-dimethyl-1,3-dioxolan und Al(O-Isopropyl)₃ (Verhältnis von Selektor : Alkohol von 2 : 1)

26,0 mmol (12,0 g) (4R,5R)-bis(Hydroxydiphenylmethyl)-2,2-dimethyl-1,3-dioxolan und 26,0 mmol (5,25 g) Aluminiumtriisopropoxid werden in einer Glovebox in 60 ml Diphenylether gegeben. Die entstandene Suspension wird bei 90 °C unter Schutzgas für 60 min gerührt. Im Anschluss werden 13 mmol (1,56 g) 1-Phenylethanol mit einem Enantiomerenüberschuss an (R)-1-Phenylethanol von 94,2 % ee zugegeben und für weitere 10 min gerührt. Anschließend wird bei etwa 20 mbär das entstandene Isopropanol abdestilliert. Danach wird bei 1 mbar und 90 °C 1,10 g eines Gemischs aus (R)-1-Phenylethanol und (S)-1-Phenylethanol als Destillat erhalten. Untersuchung des Destillats durch chirale Gaschromatographie (BGB-Analytikvertrieb BGB-174S) ergab einen Enantiomerenüberschuss an (R)-1-Phenylethanoi von 95,4 %.

### Beispiel 23:

### Trennung von rac.-trans-1,2-Diaminocyclohexan mit einem chiralen Selektor erzeugt aus (4R,5R)-bis(Hydroxydiphenylmethyl)-2,2-dimethyl-1,3-dioxolan und Ti(O-Isopropyl)₄

6,5 mmol (3,0 g) (4R,5R)-bis(Hydroxydiphenylmethyl)-2,2-dimethyl-1,3-dioxolan und 6,5 mmol (1,83 g) Titantetraisopropoxid werden in einer Glovebox in 20 ml Diphenylether gegeben. Die entstandene gelbe Lösung wird bei 90 °C unter Schutzgas für 60 min gerührt. Im Anschluss werden 26 mmol (2,94 g) racemisches trans-1,2-Diaminocyclohexan zugegeben und für weitere 10 min gerührt. Anschließend wird bei etwa 20 mbar das entstandene Isopropanol abdestilliert. Danach wird bei 1 mbar und 90 °C ca. 0,8 g eines Gemischs aus Diphenylether und 1,2-Diaminocyclohexan abdestilliert. Der gaschromatographisch (Chirasil Dex CB, TFA-Derivatisierung) bestimmte Enantiomerenüberschuss für (S,S)-1,2-trans-Diaminocyclohexan betrug 12,9 %.

### Beispiel 24:

### Trennung von rac.-1,2-Propandiol mit einem chiralen Selektor erzeugt aus (4R,5R)-bis(Hydroxydiphenylmethyl)-2,2-dimethyl-1,3-dioxolan und Ti(O-Isopropyl)₄ in Gegenwart von 2-tert-Butyl-4-methoxyphenol und 3-tert-Butyl-4-methoxyphenol

6,5 mmol (3,0 g) (4R,5R)-bis(Hydroxydiphenylmethyl)-2,2-dimethyl-1,3-dioxolan, 6,5 mmol (1,83 g) Titantetraisopropoxid und 13 mmol (2,32 g) eines Gemischs aus 2-tert-Butyl-4-methoxyphenol und 3-tert-Butyl-4-methoxyphenol werden in einer Glovebox in 20 ml Diphenylether gegeben. Die entstandene orange Lösung wird bei 90 °C unter Schutzgas für 60 min gerührt. Im Anschluss werden 13 mmol (0,99 g) rac.-1,2-Propandiol zugegeben und für weitere 10 min gerührt. Anschließend wird bei etwa 20 mbar das entstandene Isopropanol abdestilliert. Danach wird bei 1 mbar und 90 °C ca. 1,0 g eines Gemischs aus Diphenylether und 1,2-Propandiol abdestilliert. Der gaschromatographisch (BGB-174S, TFA-Dervatisierung) bestimmte Enantiomerenüberschuss für (S)-1,2-Propandiol betrug 9,2 %.

### Beispiel 25:

### Trennung von rac.-1,2-Propandiol mit einem chiralen Selektor erzeugt aus (4R,5R)-bis(Hydroxydiphenylmethyl)-2,2-dimethyl-1,3-dioxolan und Ti(O-Isopropyl)₄ in Gegenwart von. Didodecylamin

6,5 mmol (3,0 g) (4R,5R)-bis(Hydroxydiphenylmethyl)-2,2-dimethyl-1,3-dioxolan, 6,5 mmol (1,83 g) Titantetraisopropoxid und 13 mmol (4,6 g) Didodecylamin werden in einer Glovebox in 20 ml Diphenylether gegeben. Die entstandene klare Lösung wird bei 90 °C unter Schutzgas für 60 min gerührt. Im Anschluss werden 26 mmol (1,98 g) rac.-1,2-Propandiol zugegeben und für weitere 10 min gerührt. Anschließend wird bei etwa 20 mbar das entstandene Isopropanol abdestilliert. Danach wird bei 1 mbar und 90 °C ca. 3,15 g eines Gemischs aus Diphenylether und 1,2-Propandiol abdestilliert. Der gaschromatographisch (BGB-174S, TFA-Derivatisierung) bestimmte Enantiomerenüberschuss an (S)-1,2-Propandiol betrug 17,6 %.

### Beispiel 26:

### Trennung von rac.-1,2-Propandiol mit einem chiralen Selektor erzeugt aus (4R,5R)-bis(Hydroxydiphenylmethyl)-2,2-dimethyl-1,3-dioxolan und Ti(O-Isopropyl)₄ in Gegenwart von Dodecylamin

6,5 mmol (3,0 g) (4R,5R)-bis(Hydroxydiphenylmethyl)-2,2-dimethyl-1,3-dioxolan, 6,5 mmol (1,83 g) Titantetraisopropoxid und 13 mmol (2,41 g) Dodecylamin werden in einer Glovebox in 20 ml Diphenylether gegeben. Die entstandene klare Lösung wird bei 90 °C unter Schutzgas für 60 min gerührt. Im Anschluss werden 26 mmol (1,98 g) rac.-1,2-Propandiol zugegeben und für weitere 10 min gerührt. Anschließend wird bei etwa 20 mbar das entstandene Isopropanol abdestilliert. Danach werden bei 1 mbar und 90 °C 2,3 g eines Gemischs aus Diphenylether und 1,2-Propandiol abdestilliert. Der gaschromatographisch (BGB-174S, TFA-Derivatisierung) bestimmte Enantiomerenüberschuss an (S)-1,2-Propandiol betrug 14,8 %.

## Patentansprüche

1. Verfahren zur Auftrennung stereoisomerer Verbindungen, durch destillative Trennung in Gegenwart eines chiralen Diskriminators, bei dem es sich um einen Metallkomplex handelt, der wenigstens einen Liganden aufweist, der sich von einer Verbindung der allgemeinen Formel I 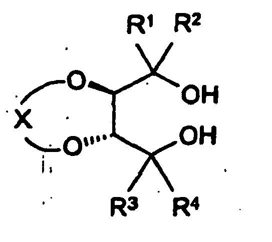 ableitet, worin
R¹, R², R³ und R⁴ unabhängig voneinander für Alkyl, Cycloalkyl, Heterocycloalkyl, Aryl oder Hetaryl stehen, wobei die Alkylreste 1, 2, 3, 4 oder 5 Substituenten, ausgewählt unter Cycloalkyl, Heterocycloalkyl, Aryl, Hetaryl, Alkoxy, Cycloalkoxy, Heterocycloalkoxy, Aryloxy, Hetaryloxy, Hydroxy, Thiol, Polyalkylenoxid, Polyalkylenimin, COOH, Carboxylat, SO₃H, Sulfonat, NE¹E², NE¹E²E³A⁻, Halogen, Nitro, Acyl oder Cyano aufweisen können, worin E¹, E² und E³ jeweils gleiche oder verschiedene Reste, ausgewählt unter Wasserstoff, Alkyl, Cycloalkyl, oder Aryl bedeuten und A⁻ für ein Anionäquivalent steht,
und wobei die Cycloalkyl-, Heterocycloalkyl-, Aryl- und Hetarylreste R¹, R², R³ und R⁴ je 1, 2, 3, 4 oder 5 Substituenten aufweisen können, die ausgewählt sind unter Alkyl und den zuvor für die Alkylreste R¹ bis R⁴ genannten Substituenten, oder
R¹ und R² und/oder R³ und R⁴ zusammen mit dem Kohlenstoffatom an das sie gebunden sind, für einen 5- bis 8-glledrigen Cyclus stehen, der gegebenenfalls zusätzlich ein-, zwei- oder dreifach mit Cycloalkyl, Heterocycloalkyl, Aryl oder Hetaryl anelliert ist, wobei der Cyclus und, falls vorhanden, die anellierten Gruppen unabhängig voneinander je einen, zwei, drei oder vier Substituenten tragen können, die ausgewählt sind unter Alkyl, Cycloalkyl, Heterocycloalkyl, Aryl, Hetaryl, Hydroxy, Thiol, Polyalkylenoxid, Polyalkylenimin, Alkoxy, Halogen, COOH, Carboxylat, SO₃H, Sulfonat, NE⁴E⁵, NE⁴E⁵E⁶A⁻, Nitro, Alkoxycarbonyl, Acyl oder Cyano stehen, worin E⁴, E⁵ und E⁶ jeweils gleiche oder verschiedene Reste, ausgewählt unter Wasserstoff, Alkyl, Cycloalkyl und Aryl bedeuten und A⁻ für ein Anionäquivalent steht,
X zusammen mit den Sauerstoffatomen, an die es gebunden ist, für einen 5- bis 8-gliedrigen Heterocyclus steht, der gegebenenfalls ein-, oder zweifach mit Cycloalkyl, Heterocycloalkyl, Aryl und/oder Hetaryl anelliert ist, wobei die anellierten Gruppen unabhängig voneinander je einen, zwei, drei oder vier Substituenten, ausgewählt unter Alkyl, Cycloalkyl, Heterocycloalkyl, Aryl, Hetaryl, Hydroxy, Thiol, Polyalkylenoxid, Polyalkylenimin, Alkoxy, Halogen, COOH, Carboxylat, SO₃H, Sulfonat, NE⁷E⁸, NE⁷E⁸E⁹A⁻, Nitro, Alkoxycarbonyl, Acyl oder Cyano, worin E⁷, E⁸ und E⁹ jeweils gleiche oder verschiedene Reste, ausgewählt unter Wasserstoff, Alkyl, Cycloalkyl und Aryl bedeuten und A⁻ für ein Anionäquivalent steht, tragen können und/oder X einen, zwei, drei oder vier Substituenten aufweisen kann, die ausgewählt sind unter den zuvor für die anellierten Gruppen genannten Substituenten und/oder X durch 1 oder 2 gegebenenfalls substituierte Heteroatome unterbrochen sein kann.

2. Verfahren nach Anspruch 1, wobei es sich bei der aufzutrennenden stereoisomeren Verbindung um einen Alkohol- handelt.

3. Verfahren nach Anspruch 2, wobei es sich bei der aufzutrennenden stereoisomeren Verbindung um einen Alkohol der Formel (III) handelt, 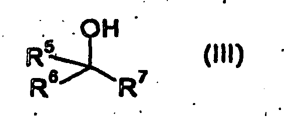 wobei
R⁵ und R⁶ unabhängig von einander ausgewählt sind unter Alkyl, Cycloalkyl Heterocycloalkyl, Aryl und Hetaryl, die jeweils gegebenenfalls substituiert sein können;
R⁷ für Wasserstoff steht oder eine der für R⁵ und R⁶ gegebenen Bedeutungen besitzt; und
wobei die Substituenten R⁵, R⁶ und R⁷ jeweils unterschiedlich voneinander sind.

4. Verfahren nach Anspruch 1, wobei es sich bei der aufzutrennenden stereoisomeren Verbindung um eine stereoisomere Verbindung mit zwei funktionellen Gruppen handelt, die ausgewählt sind unter Alkohol- und/oder Aminogruppen.

5. Verfahren nach Anspruch 4, wobei es sich bei der aufzutrennenden stereoisomeren Verbindung um eine stereoisomere Verbindung der allgemeinen Formel (IV) handelt, 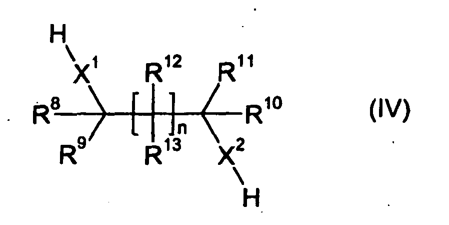 wobei
X¹ und X² unabhängig voneinander für O oder NR¹⁴ stehen, wobei R¹⁴ ausgewählt ist unter Wasserstoff, Alkyl, Cycloalkyl und Aryl, die jeweils gegebenenfalls substituiert sein können;
n für 0 oder 1 steht; und
wobei unter der Bedingung, dass R⁸ und R⁹ voneinander unterschiedlich sind und/oder R¹⁰ und R¹¹ voneinander unterschiedlich sind und/oder R¹² und R¹³ wenn vorhanden voneinander unterschiedlich sind
R⁸, R⁹, R¹⁰, R¹¹ unabhängig voneinander ausgewählt sind unter Wasserstoff, Alkyl, Cycloalkyl, Heterocycloalkyl, Aryl und Hetaryl, wobei die letzten drei Radikale jeweils gegebenenfalls substituiert sein können; oder
R⁸ zusammen mit R¹⁰ und den Kohlenstoffatomen, über die diese miteinander verbunden sind, einen 3- bis 8-gliedrigen, gesättigten oder teilweise ungesättigten Carbocyclus bilden, der gegebenenfalls substituiert ist und weitere anellierte gesättigte, teilweise ungesättigte oder aromatische Carbocyclen oder Heterocyclen umfassen kann; oder
wenn X¹ und/oder X² für NR¹⁴ steht, R⁸ zusammen mit R¹⁴ und/oder R¹⁰ zusammen mit R¹⁴ und jeweils zusammen mit den Atomen, über die diese miteinander verbunden sind, einen gegebenenfalls substituierten, stickstoffhaltigen Ring bilden können;
R¹² und R¹³ unabhängig voneinander.eine der für R⁸, R⁹, R¹⁰, R¹¹ gegebenen Bedeutungen besitzt; oder .
R¹² zusammen mit R⁸ und den Kohlenstoffatomen, über die diese miteinander verbunden sind, einen 3- bis 8-gliedrigen, gesättigten oder teilweise ungesättigten Carbocyclus bilden, der gegebenenfalls substituiert ist und weitere anellierte gesättigte, teilweise ungesättigte oder aromatische Carbocyclen oder Heterocyclen umfassen kann; oder
wenn X¹ und/oder X² für NR¹⁴ steht, R¹² zusammen mit R¹⁴ und/oder R¹³ zusammen mit R¹⁴ und jeweils zusammen mit den Atomen, über die diese miteinander verbunden sind, einen gegebenenfalls substituierten, stickstoffhaltigen Ring bilden können.

6. Verfahren nach einem der Ansprüche 2 bis 5, wobei der als chiraler Diskriminator eingesetzte Metallkomplex ausgewählt wird aus Verbindungen der allgemeinen Formel I.b 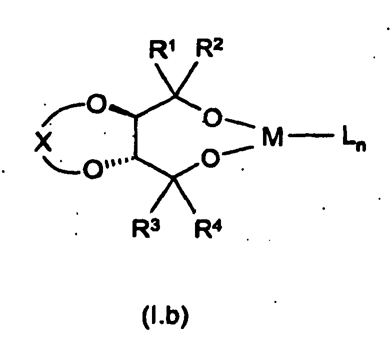 worin
R¹, R², R³, R⁴ und X die in Anspruch 1 angegebenen Bedeutungen besitzen,
M für ein Metall, vorzugsweise Al oder Ti steht,
n für 1 oder 2 steht, und
L unabhängig voneinander ausgewählt ist unter Alkyloxy, Cycloalkyloxy, Aryloxy, Arylalkyloxy, Alkylthio, Cycloalkylthio, Arylthio, Arylalkylthio, N,N-Dialkylamino, N,N-Dicycloalkylamino, N,N-Diarylamino, N,N-Diarylalkylamino, N-Aryl-N-alkylamino, N-Aryl-N-arylaikylamino, Alkyl, Cycloalkyl, Aryl oder Arylalkyl.

7. Verfahren nach Anspruch 6, wobei sich L von den zur Auftrennung eingesetzten stereoisomeren Alkoholen ableitet.

8. Verfahren nach Anspruch 6, wobei M für ein dreiwertiges Metall steht und es sich bei der aufzutrennenden stereoisomeren Verbindung um einen Alkohol handelt.

9. Verfahren nach Anspruch 6, wobei M für ein vierwertiges Metall steht und es sich bei der aufzutrennenden stereoisomeren Verbindung um eine stereoisomere Verbindung mit zwei funktionellen Gruppen handelt, die ausgewählt sind unter Alkohol- und/oder Aminogruppen.

10. Verfahren nach einem der Ansprüche 2, 3 oder 6, wobei es sich bei den stereoisomeren Verbindungen, speziell Alkoholen, um Enantiomere handelt.

11. Verfahren nach einem der Ansprüche 3 oder 5 bis 6, wobei es sich bei der stereoisomere Verbindung mit zwei funktionellen Gruppen, die ausgewählt sind unter Alkohol- und/oder Aminogruppen, um Enantiomere oder Diastereomere handelt.

12. Verfahren nach einem der vorhergehenden Ansprüche, wobei es sich bei der destillativen Trennung um eine Extraktivdestillation, vorzugsweise um eine mehrstufige Extraktivdestillation handelt.

13. Verfahren nach Anspruch 12, wobei zur Auftrennung ein zusätzliches Lösungsmittel eingesetzt wird, das einen höheren Siedepunkt als die aufzutrennenden stereoisomeren Verbindungen aufweist.

14. Verfahren nach Anspruch 13, wobei das zur Auftrennung eingesetzte zusätzliche Lösungsmittel bei dem Druck, unter dem die Auftrennung erfolgt, einen Siedepunkt aufweist, der um wenigstens 5 °C, besonders bevorzugt um wenigstens 10 °C, höher liegt, als der Siedepunkt des am höchsten siedenden Stereoisomers.

## Claims

1. A method for fractionating stereoisomeric compounds by distillative separation in the presence of a chiral discriminator which is a metal complex having at least one ligand which is derived from a compound of the general formula I 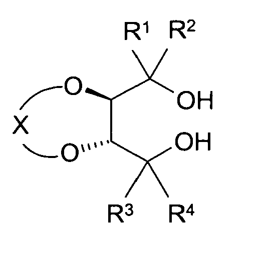 in which
R¹, R², R³ and R⁴ are independently of one another alkyl, cycloalkyl, heterocycloalkyl, aryl or hetaryl, where the alkyl radicals may have 1, 2, 3, 4 or 5 substituents selected from cycloalkyl, heterocycloalkyl, aryl, hetaryl, alkoxy, cycloalkoxy, heterocycloalkoxy, aryloxy, hetaryloxy, hydroxy, thiol, polyalkylene oxide, polyalkyleneimine, COOH, carboxylate, SO₃H, sulfonate, NE¹E², NE¹E²E³A⁻, halogen, nitro, acyl or cyano, in which E¹, E² and E³ are in each case identical or different radicals selected from hydrogen, alkyl, cycloalkyl, or aryl, and A⁻ is an anion equivalent,
and where the cycloalkyl, heterocycloalkyl, aryl and hetaryl radicals R¹, R², R³ and R⁴ may each have 1, 2, 3, 4 or 5 substituents which are selected from alkyl and the substituents mentioned above for the alkyl radicals R¹ to R⁴, or
R¹ and R² and/or R³ and R⁴ together with the carbon atom to which they are bonded are a 5- to 8-membered ring which is optionally additionally fused one, two or three times to cycloalkyl, heterocycloalkyl, aryl or hetaryl, where the ring and, if present, the fused groups may independently of one another each have one, two, three or four substituents which are selected from alkyl, cycloalkyl, heterocycloalkyl, aryl, hetaryl, hydroxy, thiol, polyalkylene oxide, polyalkyleneimine, alkoxy, halogen, COOH, carboxylate, SO₃H, sulfonate, NE⁴E⁵, NE⁴E⁵E⁶A⁻, nitro, alkoxycarbonyl, acyl or cyano, in which E⁴, E⁵ and E⁶ are in each case identical or different radicals selected from hydrogen, alkyl, cycloalkyl and aryl, and A⁻ is an anion equivalent,
X together with the oxygen atoms to which it bonded is a 5- to 8-membered heterocycle which is optionally fused once or twice to a cycloalkyl, heterocycloalkyl, aryl and/or hetaryl, where the fused groups may independently of one another each have one, two, three or four substituents selected from alkyl, cycloalkyl, heterocycloalkyl, aryl, hetaryl, hydroxy, thiol, polyalkylene oxide, polyalkyleneimine, alkoxy, halogen, COOH, carboxylate, SO₃H, sulfonate, NE⁷E⁸, NE⁷E⁸E⁹A⁻, nitro, alkoxycarbonyl, acyl or cyano, in which E⁷, E⁸ and E⁹ are in each case identical or different radicals selected from hydrogen, alkyl, cycloalkyl and aryl, and A⁻ is an anion equivalent, and/or X may have one, two, three or four substituents which are selected from the substituents mentioned above for the fused groups, and/or X may be interrupted by 1 or 2 optionally substituted heteroatoms.

2. The method according to claim 1, where the stereoisomeric compound to be fractionated is an alcohol.

3. The method according to claim 2, where the stereoisomeric compound to be fractionated is an alcohol of the formula (III) 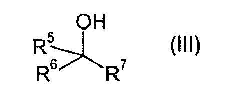 where
R⁵ and R⁶ are independently of one another selected from alkyl, cycloalkyl, heterocycloalkyl, aryl and hetaryl, each of which may optionally be substituted;
R⁷ is hydrogen or has one of the meanings given for R⁵ and R⁶; and
where the substituents R⁵, R⁶ and R⁷ are each different from one another.

4. The method according to claim 1, where the stereoisomeric compound to be fractionated is a stereoisomeric compound having two functional groups which are selected from alcohol and/or amino groups.

5. The method according to claim 4, where the stereoisomeric compound to be fractionated is a stereoisomeric compound of the general formula (IV) 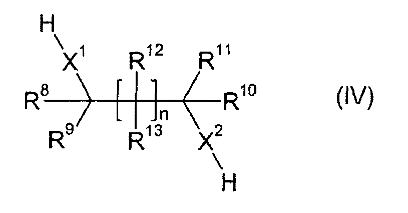 where
X¹ and X² are independently of one another O or NR¹⁴, where R¹⁴ is selected from hydrogen, alkyl, cycloalkyl and aryl, each of which may optionally be substituted;
n is 0 or 1; and
where with the proviso that R⁸ and R⁹ are different from one another, and/or R¹⁰ and R¹¹ are different from one another, and/or R¹² and R¹³ if present are different from one another,
R⁸, R⁹, R¹⁰, R¹¹ are selected independently of one another from hydrogen, alkyl, cycloalkyl, heterocycloalkyl, aryl and hetaryl, where each of the last three radicals may optionally be substituted; or
R⁸ together with R¹⁰ and the carbon atoms via which they are linked together form a 3- to 8-membered, saturated or partly unsaturated carbocycle which is optionally substituted and may comprise further fused saturated, partly unsaturated or aromatic carbocycles or heterocycles; or
if X¹ and/or X² is NR¹⁴, then R⁸ together with R¹⁴ and/or R¹⁰ together with R¹⁴ and in each case together with the atoms via which they are linked together may form an optionally substituted nitrogen-containing ring;
R¹² and R¹³ has independently of one another one of the meanings given for R⁸, R⁹, R¹⁰, R¹¹_{;} or
R¹² together with R⁸ and the carbon atoms via which they are linked together form a 3- to 8-membered, saturated or partly unsaturated carbocycle which is optionally substituted and may comprise further fused saturated, partly unsaturated or aromatic carbocycles or heterocycles; or
if X¹ and/or X² is NR¹⁴, then R¹² together with R¹⁴ and/or R¹³ together with R¹⁴ and in each case together with the atoms, via which they are linked together may form an optionally substituted, nitrogen-containing ring.

6. The method according to any of claims 2 to 5, where the metal complex employed as chiral discriminator is selected from compounds of the general formula I.b 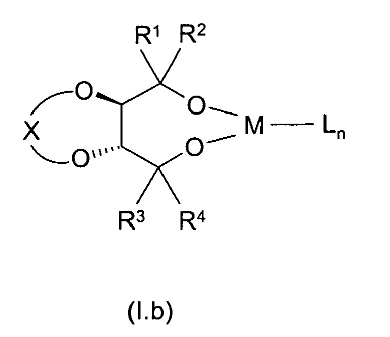 in which
R¹, R², R³, R⁴ and X have the meanings indicated in claim 1,
M is a metal, preferably Al or Ti,
n is 1 or 2, and
L is selected independently of one another from alkyloxy, cycloalkyloxy, aryloxy, arylalkyloxy, alkylthio, cycloalkylthio, arylthio, arylalkylthio, N,N-dialkylamino, N,N-dicycloalkylamino, N,N-diarylamino, N,N-diarylalkylamino, N-aryl-N-alkylamino, N-aryl-N-arylalkylamino, alkyl, cycloalkyl, aryl or arylalkyl.

7. The method according to claim 6, where L is derived from the stereoisomeric alcohols employed for the fractionation.

8. The method according to claim 6, where M is a trivalent metal, and the stereoisomeric compond to be fractionated is an alcohol.

9. The method according to claim 6, where M is a tetravalent metal, and the stereoisomeric compound to be fractionated is a stereoisomeric compound having two functional groups which are selected from alcohol and/or amino groups.

10. The method according to any of claims 2, 3 or 6, where the stereoisomeric compounds, specifically alcohols, are enantiomers.

11. The method according to any of claims 3 or 5 to 6, where the stereoisomeric compound having two functional groups which are selected from alcohol and/or amino groups comprises enantiomers or diastereomers.

12. The method according to any of the preceding claims, where the distillative separation comprises an extractive distillation, preferably a multistage extractive distillation.

13. The method according to claim 12, where an additional solvent which has a higher boiling point than the stereoisomeric compounds to be fractionated is employed for the fractionation.

14. The method according to claim 13, where the additional solvent employed for the fractionation has a boiling point at the pressure under which the fractionation takes place which is at least 5°C, particularly preferably at least 10°C, higher than the boiling point of the highest-boiling stereoisomer.

## Revendications

1. Procédé de séparation de composés stéréo-isomères, par séparation par distillation en présence d'un agent de discrimination chiral, où il s'agit d'un complexe métallique qui présente au moins un ligand, qui est dérivé d'un composé de formule générale I 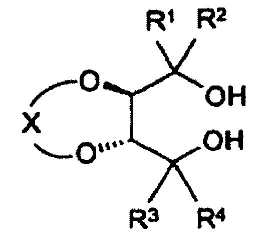 dans laquelle
R¹, R², R³ et R⁴ indépendamment l'un de l'autre, représentent alkyle, cycloalkyle, hétérocycloalkyle, aryle ou hétaryle, où les radicaux alkyle peuvent présenter 1, 2, 3, 4 ou 5 substituants choisis parmi cycloalkyle, hétérocycloalkyle, aryle, hétaryle, alcoxy, cycloalcoxy, hétérocycloalcoxy, aryloxy, hétaryloxy, hydroxy, thiol, poly(oxyde d'alkylène), polyalkylénimine, COOH, carboxylate, SO₃H, sulfonate, NE¹E², NE¹E²E³A⁻, halogène, nitro, acyle ou cyano, où E¹, E² et E³ signifient des radicaux à chaque fois identiques ou différents, choisis parmi hydrogène, alkyle, cycloalkyle, ou aryle et A⁻ représente un équivalent d'anion,
et où les radicaux cycloalkyle, hétérocycloalkyle, aryle et hétaryle R¹, R², R³ et R⁴ peuvent présenter chacun 1, 2, 3, 4 ou 5 substituants, qui sont choisis parmi alkyle et les substituants mentionnés ci-dessus pour les radicaux alkyle R¹ à R⁴, ou
R¹ et R² et/ou R³ et R⁴ ensemble avec l'atome de carbone auquel ils sont liés, représentent un cycle de 5 à 8 chaînons, qui est le cas échéant en outre monocondensé, dicondensé ou tricondensé avec cycloalkyle, hétérocycloalkyle, aryle ou hétaryle le cycle et, le cas échéant, les groupes condensés pouvant porter, indépendamment l'un de l'autre, chacun un, deux, trois ou quatre substituants qui sont choisis parmi alkyle, cycloalkyle, hétérocycloalkyle, aryle, hétaryle, hydroxy, thiol, poly(oxyde d'alkylène), polyalkylénimine, alcoxy, halogène, COOH, carboxylate, SO₃H, sulfonate, NE⁴E⁵, NE⁴E⁵E⁶A⁻, nitro, alcoxycarbonyle, acyle ou cyano, où E⁴, E⁵ et E⁶ signifient des radicaux à chaque fois identiques ou différents, choisis parmi hydrogène, alkyle, cycloalkyle et aryle et A⁻ représente un équivalent d'anion,
X ensemble avec l'atome d'oxygène, auquel il est lié, représente un hétérocycle de 5 à 8 chaînons, qui est le cas échéant monocondensé ou dicondensé avec cycloalkyle, hétérocycloalkyle, aryle et/ou hétaryle, les groupes condensés pouvant porter indépendamment l'un de l'autre chacun un, deux, trois ou quatre substituants, choisis parmi alkyle, cycloalkyle, hétérocycloalkyle, aryle, hétaryle, hydroxy, thiol, poly(oxyde d'alkylène), polyalkylénimine, alcoxy, halogène, COOH, carboxylate, SO₃H, sulfonate, NE⁷E⁸, NE⁷E⁸E⁹A⁻, nitro, alcoxycarbonyle, acyle ou cyano, où E⁷, E⁸ et E⁹ signifient des radicaux à chaque fois identiques ou différents, choisis parmi hydrogène, alkyle, cycloalkyle et aryle et A⁻ représente un équivalent d'anion et/ou X peut représenter un, deux, trois ou quatre substituants qui sont choisis parmi les substituants mentionnés ci-dessus pour les groupes condensés et/ou X peut être interrompu par 1 ou 2 hétéroatomes le cas échéant substitués.

2. Procédé selon la revendication 1, où il s'agit, pour le composé stéréo-isomère à séparer, d'un alcool.

3. Procédé selon la revendication 2, où il s'agit, pour le composé stéréo-isomère à séparer, d'un alcool de formule (III), 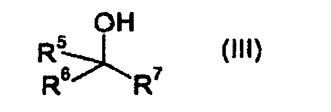 où
R⁵ et R⁶ indépendamment l'un de l'autre, sont choisis parmi alkyle, cycloalkyle hétérocycloalkyle, aryle et hétaryle, qui peuvent à chaque fois, le cas échéant, être substitués ;
R⁷ représente hydrogène ou présente une des significations indiquées pour R⁵ et R⁶ ; et
où les substituants R⁵, R⁶ et R⁷ sont à chaque fois différents les uns des autres.

4. Procédé selon la revendication 1, où il s'agit, pour le composé stéréo-isomère à séparer, d'un composé stéréo-isomère présentant deux groupes fonctionnels qui sont choisis parmi les groupes alcool et/ou amino.

5. Procédé selon la revendication 4, où il s'agit, pour le composé stéréo-isomère à séparer, d'un composé stéréo-isomère de formule générale (IV), 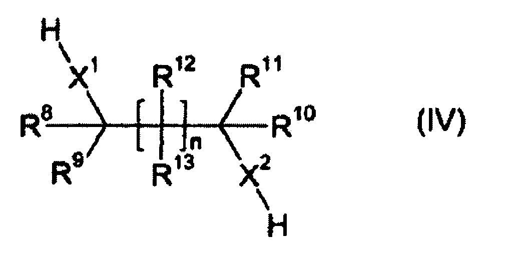 où
X¹ et X² indépendamment l'un de l'autre, représentent O ou NR¹⁴, où R¹⁴ est choisi parmi hydrogène, alkyle, cycloalkyle et aryle, qui peuvent à chaque fois, le cas échéant, être substitués ;
n vaut 0 ou 1 ; et
où, dans la condition dans laquelle R⁸ et R⁹ sont différents l'un de l'autre et/ou R¹⁰ et R¹¹ sont différents l'un de l'autre et/ou R¹² et R¹³ sont, le cas échéant, différents l'un de l'autre
R⁸, R⁹, R¹⁰, R¹¹ indépendamment l'un de l'autre, sont choisis parmi hydrogène, alkyle, cycloalkyle hétérocycloalkyle, aryle et hétaryle, les trois derniers radicaux pouvant à chaque fois, le cas échéant, être substitués ; ou
R⁸ ensemble avec R¹⁰ et les atomes de carbone via lesquels ceux-ci sont liés l'un à l'autre, forment un carbocycle de 3 à 8 chaînons, saturé ou partiellement insaturé, qui est le cas échéant substitué et qui peut comprendre d'autres carbocycles ou hétérocycles condensés, saturés, partiellement insaturés ou aromatiques ; ou
lorsque X¹ et/ou X² représente NR¹⁴, R⁸, ensemble avec R¹⁴ et/ou R¹⁰ ensemble avec R¹⁴ et à chaque fois ensemble avec les atomes via lesquels ceux-ci sont liés l'un à l'autre, peuvent former un cycle azoté, le cas échéant substitué ;
R¹² et R¹³ , indépendamment l'un de l'autre, présentent une des significations indiquées pour R⁸, R⁹, R¹⁰, R¹¹ _{;} ou
R¹² ensemble avec R⁸ et les atomes de carbone via lesquels ceux-ci sont liés l'un à l'autre, forment un carbocycle de 3 à 8 chaînons, saturé ou partiellement insaturé, qui est le cas échéant substitué et qui peut comprendre d'autres carbocycles ou hétérocycles condensés, saturés, partiellement insaturés ou aromatiques ; ou
lorsque X¹ et/ou X² représente NR¹⁴, R¹², ensemble avec R¹⁴ et/ou R¹³ ensemble avec R¹⁴ et à chaque fois ensemble avec les atomes via lesquels ceux-ci sont liés l'un à l'autre, peuvent former un cycle azoté, le cas échéant substitué.

6. Procédé selon l'une quelconque des revendications 2 à 5, où on choisit le complexe métallique utilisé comme agent de discrimination chiral parmi les composés de formule générale I.b 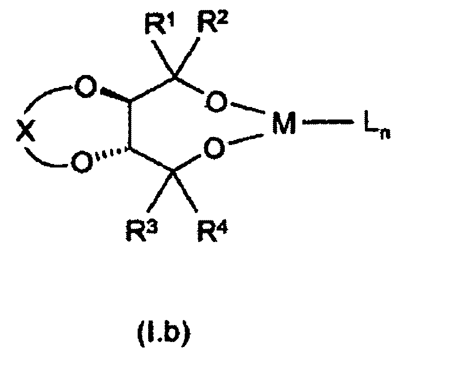 où
R¹, R² R³, R⁴ et X présentent les significations indiquées dans la revendication 1,
M représente un métal, de préférence Al ou Ti,
n vaut 1 ou 2 ; et
L indépendamment l'un de l'autre, est choisi parmi alkyloxy, cycloalkyloxy, aryloxy, arylalkyloxy, alkylthio, cycloalkylthio, arylthio, arylalkylthio, N,N-dialkylamino, N,N-dicycloalkylamino, N,N-diarylamino, N,N-diarylalkylamino, N-aryl-N-alkylamino, N-aryl-N-arylalkylamino, alkyle, cycloalkyle, aryle ou arylalkyle.

7. Procédé selon la revendication 6, où L est dérivé des alcools stéréo-isomères utilisés pour la séparation.

8. Procédé selon la revendication 6, où M représente un métal trivalent et où il s'agit, pour le composé stéréo-isomère à séparer, d'un alcool.

9. Procédé selon la revendication 6, où M représente un métal tétravalent et où il s'agit, pour le composé stéréo-isomère à séparer, d'un composé stéréo-isomère présentant deux groupes fonctionnels qui sont choisis parmi les groupes alcool et/ou amino.

10. Procédé selon l'une quelconque des revendications 2, 3 ou 6, où il s'agit, pour les composés stéréo-isomères, en particulier pour les alcools, d'énantiomères.

11. Procédé selon l'une quelconque des revendications 3 ou 5 à 6, où il s'agit, pour le composé stéréo-isomère présentant deux groupes fonctionnels qui sont choisis parmi les groupes alcool et/ou amino, d'énantiomères ou de diastéréo-isomères.

12. Procédé selon l'une quelconque des revendications précédentes, où il s'agit, pour la séparation par distillation, d'une distillation avec extraction, de préférence d'une distillation avec extraction à plusieurs étapes.

13. Procédé selon la revendication 12, où on utilise pour la séparation un solvant supplémentaire qui présente un point d'ébullition supérieur à celui des composés stéréo-isomères à séparer.

14. Procédé selon la revendication 13, où le solvant supplémentaire utilisé pour la séparation, à la pression à laquelle la séparation s'effectue, présente un point d'ébullition qui est supérieur d'au moins 5°C, de manière particulièrement préférée d'au moins 10°C au point d'ébullition du stéréo-isomère présentant le point d'ébullition le plus élevé.
